# EUROPEAN PATENT APPLICATION

(11) **EP 4 205 763 A1**
(43) Date of publication of application: **05.07.2023**
(21) Application number: 21860461.9
(22) Date of filing: 26.08.2021
(51) Int. Cl.: A61K 39/395, A61P 35/00, C07K 16/00

(54) **USE OF ANTI-PD-1 ANTIBODY IN TREATMENT OF NASOPHARYNGEAL CARCINOMA**

(30) Priority: 27.08.2020 CN 202010879644; 30.03.2021 CN 202110343389; 12.08.2021 CN 202110926736
(71) Applicant: Shanghai Junshi Biosciences Co., Ltd., Pilot Free Trade Zone Shanghai 201210 (CN)
(72) Inventor: YAO, Sheng, Shanghai 201210 (CN); FENG, Hui, Shanghai 201210 (CN)
(74) Representative: V.O.
(86) International application number: PCT/CN2021/114668
(87) International publication number: WO 2022/042626

(57) **Abstract**

The present invention relates to the use of an anti-PD-1 antibody or an antigen-binding fragment thereof in preparing a drug for preventing or treating a malignant cancer, and the use of a combination of an anti-PD-1 antibody or an antigen-binding fragment thereof and gemcitabine-cisplatin in preparing a drug for preventing or treating a malignant cancer. The malignant cancer is preferably nasopharyngeal carcinoma. The present invention also relates to a method for using a biomarker to predict the therapeutic effect of the anti-PD-1 antibody or the antigen-binding fragment thereof in treatment of nasopharyngeal carcinoma.

## Description

### Technical Field

The present invention relates to the use of an anti-PD-1 antibody or an antigen-binding fragment thereof in treatment of a malignant cancer. In particular, the present invention relates to the use of an anti-PD-1 antibody or an antigen-binding fragment thereof in preparing a drug for treating nasopharyngeal carcinoma, the use of a combination of an anti-PD-1 antibody or an antigen-binding fragment thereof and gemcitabine-cisplatin in preparing a drug for preventing or treating a malignant cancer, and a method for using a biomarker to predict the therapeutic effect of the anti-PD-1 antibody or the antigen-binding fragment thereof in treatment of nasopharyngeal carcinoma.

### Background Art

Immune escape is one of the characteristics of cancer. Ahmadzadeh, M. et al disclosed in Blood, 114: 1537-44 that tumour-specific T lymphocytes are often present in the tumour microenvironment, draining lymph nodes and peripheral blood, but are generally unable to control tumour progression due to the network of immunosuppressive mechanisms present in the tumour microenvironment. CD8⁺ tumour infiltrating lymphocytes (TILs) generally express activation-induced inhibitory receptors, including CTLA-4 and PD-1, while tumour cells often express immunosuppressive ligands, including PD-1 ligand 1 (PD-L1, also called B7-H1 or CD274) which inhibits activation and effector functions of T cells. In the inhibitory mechanism, PD-1 and its ligands have become an important pathway for tumour cells to suppress activated T cells in the tumour microenvironment.

Programmed death receptor 1 (PD-1) plays an important role in immune regulation and maintenance of peripheral tolerance. PD-1 is expressed primarily in activated T and B cells and functions to inhibit the activation of lymphocytes, which is a normal peripheral tissue tolerance mechanism of the immune system that prevents over-reactive immunity. However, the activated T cells infiltrated in the tumour microenvironment highly express PD-1 molecules, and the inflammatory factors secreted by the activated leukocytes may induce tumour cells to highly express ligands PD-L1 and PD-L2 of PD-1, resulting in the continuous activation of the PD-1 pathway of the activated T cells in the tumour microenvironment, the suppression of T cell function, and the inability to kill tumour cells. Therapeutic anti-PD-1 antibodies can block this pathway, partially restore the function of T cells, and enable the activated T cells to continuously kill tumour cells.

Blocking the PD-1/PD-L1 pathway has proven to be an effective way to induce a durable anti-tumour response in various cancer indications over the last decade. Monoclonal antibodies (mAbs) blocking the PD/PD-L1 pathway can enhance activation and effector functions of tumour-specific T cells, reduce tumour burden, and improve survival rate. Between 2014 and 2017, the FDA has approved two anti-PD1 monoclonal antibodies (nivolumab and pembrolizumab) and three anti-PD-L1 monoclonal antibodies (atezolizumab, avelumab and durvalumab) for treating human tumours.

Nasopharyngeal carcinoma (NPC) refers to a malignant cancer that occurs in the top and side walls of the nasopharyngeal cavity, and its incidence rate is the first among otorhinolaryngological malignant cancers. According to the World Health Organization survey, 80% of nasopharyngeal carcinoma patients in the world are in China. Because nasopharyngeal carcinoma has an insidious onset and a strong tendency to metastasize, about 75% of patients have reached an advanced stage at the first diagnosis, with local lymph node metastasis and/or distant metastasis. Generally, radiotherapy-based comprehensive treatment is very effective for early nasopharyngeal carcinoma, while recurrence or metastasis after treatment has a very poor prognosis, which is the main reason for the failure of treatment and the decline of survival rate of nasopharyngeal carcinoma. Epstein-Barr virus (EBV) infection is critical to the development of NPC. According to WHO classification, nasopharyngeal carcinoma has three histopathological types: keratinizing (type I), non-keratinizing (type II) and basal squamous cell carcinoma (type III).

However, some such antibodies that have been marketed still have some safety problems such as adverse drug events. Therefore, there remains highly unmet clinical needs for effective treatment of malignant cancers (such as nasopharyngeal carcinoma).

### Summary of the Invention

The present invention provides the use of an anti-PD-1 antibody or an antigen-binding fragment thereof in preparing a drug for preventing or treating a malignant cancer patient, and the use of a combination of an anti-PD-1 antibody or an antigen-binding fragment thereof and gemcitabine-cisplatin in preparing a drug for preventing or treating a malignant cancer.

In another aspect, the present invention provides a method for preventing or treating a malignant cancer, which comprises administering an effective amount of the anti-PD-1 antibody or the antigen-binding fragment thereof of the present invention, or a combination of the anti-PD-1 antibody or the antigen-binding fragment thereof and gemcitabine-cisplatin, to an individual in need thereof.

In another aspect, the present invention provides an anti-PD-1 antibody or an antigen-binding fragment thereof, or a combination of an anti-PD-1 antibody or an antigen-binding fragment thereof and gemcitabine-cisplatin, for use in treating or preventing a malignant cancer. In one or more embodiments, the malignant cancer of the present invention is nasopharyngeal carcinoma.

In one or more embodiments, the malignant cancer of the present invention is recurrent or metastatic nasopharyngeal carcinoma.

In one or more embodiments, the malignant cancer of the present invention is a malignant cancer with PD-L1 expression > 1% in a cancer tissue section by immunohistochemical staining analysis. As a preferred embodiment, the nasopharyngeal carcinoma of the present invention is a malignant cancer with PD-L1 expression > 25% in a cancer tissue section by analysis.

In one or more embodiments, the malignant cancer of the present invention is selected from keratinizing nasopharyngeal carcinoma and non-keratinizing nasopharyngeal carcinoma, preferably keratinizing nasopharyngeal carcinoma.

In one or more embodiments, the nasopharyngeal carcinoma of the present invention is a nasopharyngeal carcinoma in which no genome amplification of *CCND1*, *FGF14*, *FGF3* or *FGF4* chromosome 11q13 region is detected in peripheral blood circulating tumour DNA or in a tumour tissue, or the patient is a patient with nasopharyngeal carcinoma in which no genome amplification of *CCND1*, *FGF14*, *FGF3* or *FGF4* chromosome 11q13 region is detected in peripheral blood circulating tumour DNA or in a tumour tissue.

In one or more embodiments, the patient is a nasopharyngeal carcinoma patient who is refractory to standard systemic therapy, or has progressive disease 6 months after radiotherapy and chemotherapy.

In one or more embodiments, the nasopharyngeal carcinoma of the present invention is a nasopharyngeal carcinoma in which there is at least 2-fold decrease in the number of copies of EBV DNA in peripheral blood on day 28 of treatment relative to before administration on day 0.

In one or more embodiments, the anti-PD-1 antibody or the antigen-binding fragment thereof of the present invention comprises light chain complementarity determining regions with amino acid sequences as set forth in SEQ ID NOs: 1, 2 and 3, and heavy chain complementarity determining regions with amino acid sequences as set forth in SEQ ID NOs: 4, 5 and 6.

In one or more embodiments, the anti-PD-1 antibody or the antigen-binding fragment thereof of the present invention comprises a light chain variable region with an amino acid sequence as set forth in SEQ ID NO: 7, and a heavy chain variable region with an amino acid sequence as set forth in SEQ ID NO: 8.

In one or more embodiments, the anti-PD-1 antibody of the present invention comprises a light chain with an amino acid sequence as set forth in SEQ ID NO: 9, and a heavy chain with an amino acid sequence as set forth in SEQ ID NO: 10.

In one or more embodiments, the anti-PD-1 antibody of the present invention is selected from one or more of nivolumab, pembrolizumab, toripalimab, sintilimab, camrelizumab, tislelizumab and cemiplimab, preferably toripalimab.

In one or more embodiments, the anti-PD-1 antibody of the present invention is a monoclonal antibody or an antigen-binding fragment thereof.

In one or more embodiments, the anti-PD-1 antibody or the antigen-binding fragment thereof of the present invention is administered alone.

In one or more embodiments, the anti-PD-1 antibody or the antigen-binding fragment thereof of the present invention is administered at a dose from about 0.1 mg/kg body weight to about 10.0 mg/kg body weight of an individual, such as about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight of an individual, or at a dose selected from a fixed dose of about 120 mg to about 480 mg, such as a fixed dose of 120 mg, 240 mg, 360 mg or 480 mg, preferably 3 mg/kg body weight of an individual or a fixed dose of 240 mg.

In one or more embodiments, the anti-PD-1 antibody or the antigen-binding fragment thereof of the present invention is administered at a frequency of about once every week, once every two weeks, once every three weeks, once every four weeks or once a month, preferably once every two weeks.

In one or more embodiments, the anti-PD-1 antibody or the antigen-binding fragment thereof of the present invention is administered at a dose of 1 mg/kg body weight of an individual, 3 mg/kg body weight of an individual or 10 mg/kg body weight of an individual, or at a fixed dose of 240 mg or 480 mg, once every two weeks or once every three weeks.

In one or more embodiments, the anti-PD-1 antibody or the antigen-binding fragment thereof of the present invention is administered parenterally, e.g., by intravenous infusion, in a liquid dosage form, e.g., an injection.

In one or more embodiments, the anti-PD-1 antibody or the antigen-binding fragment thereof of the present invention is administered in a period of one week, two weeks, three weeks, one month, two months, three months, four months, five months, half a year or longer; and optionally, each administration period lasts for the identical or different period of time and is at identical or different intervals.

In one or more embodiments, the anti-PD-1 antibody or the antigen-binding fragment thereof of the present invention is administered in combination with gemcitabine and cisplatin.

In one or more embodiments, the anti-PD-1 antibody or the antigen-binding fragment thereof of the present invention is administered at a dose from about 0.1 mg/kg body weight to about 10.0 mg/kg body weight of an individual, such as about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight of an individual, or at a dose selected from a fixed dose of about 120 mg to about 480 mg, such as a fixed dose of 120 mg, 240 mg, 360 mg or 480 mg, preferably 3 mg/kg body weight of an individual or a fixed dose of 240 mg; and

the gemcitabine is administered at a single dose from about 600 mg/m² body surface area to about 1400 mg/m² body surface area, such as 800 mg/m² body surface area, 1000 mg/m² body surface area or 1200 mg/m² body surface area; and
the cisplatin is administered at a single dose from about 40 mg/m² body surface area to about 120 mg/m² body surface area, such as 60 mg/m² body surface area, 80 mg/m² body surface area or 100 mg/m² body surface area.

In one or more embodiments, the anti-PD-1 antibody or the antigen-binding fragment thereof of the present invention is administered at a frequency of about once every week, once every two weeks, once every three weeks, once every four weeks or once a month, preferably once every three weeks; the gemcitabine is administered at a frequency of about once every week, once every two weeks, once every three weeks, twice every three weeks, once every four weeks or once a month, preferably twice every three weeks; and the cisplatin is administered at a frequency of about once every week, once every two weeks, once every three weeks, once every four weeks or once a month, preferably once every three weeks.

In one or more embodiments, the anti-PD-1 antibody or the antigen-binding fragment thereof of the present invention is administered at a fixed dose of 240 mg, once every three weeks; and the gemcitabine is administered at a single dose of about 1000 mg/m² body surface area, twice every three weeks; and the cisplatin is administered at a single dose of about 80 mg/m² body surface area, once every three weeks.

In one or more embodiments, the anti-PD-1 antibody or the antigen-binding fragment thereof of the present invention, gemcitabine and cisplatin are administered parenterally, e.g., by intravenous infusion, in a liquid dosage form, e.g., an injection.

In one or more embodiments, the anti-PD-1 antibody or the antigen-binding fragment thereof of the present invention, gemcitabine and cisplatin are administered in periods of one week, two weeks, three weeks, one month, two months, three months, four months, five months, half a year or longer, respectively; and optionally, each administration period lasts for the identical or different period of time and is at identical or different intervals.

In another aspect, the present invention provides a drug combination comprising an anti-PD-1 antibody or an antigen-binding fragment thereof, gemcitabine and cisplatin.

In one or more embodiments, the anti-PD-1 antibody or the antigen-binding fragment thereof of the present invention comprises light chain complementarity determining regions with amino acid sequences as set forth in SEQ ID NOs: 1, 2 and 3, and heavy chain complementarity determining regions with amino acid sequences as set forth in SEQ ID NOs: 4, 5 and 6; preferably, the anti-PD-1 antibody or the antigen-binding fragment thereof comprises a light chain variable region with an amino acid sequence as set forth in SEQ ID NO: 7, and a heavy chain variable region with an amino acid sequence as set forth in SEQ ID NO: 8; preferably, the anti-PD-1 antibody comprises a light chain with an amino acid sequence as set forth in SEQ ID NO: 9, and a heavy chain with an amino acid sequence as set forth in SEQ ID NO: 10; and more preferably, the anti-PD-1 antibody is toripalimab.

In another aspect, the present invention provides the use of a reagent for detecting a gene mutation or amplification of *CCND1*, *FGF14*, *FGF3* or *FGF4* chromosome 11q13 region in peripheral blood circulating tumour DNA and/or in a tumour tissue of an individual in preparing a kit for predicting a therapeutic effect of an anti-PD-1 antibody on nasopharyngeal carcinoma.

In another aspect, the present invention provides the use of a reagent for detecting the number of copies of EBV DNA in peripheral blood of an individual in preparing a kit for predicting the therapeutic effect of an anti-PD-1 antibody on nasopharyngeal carcinoma.

In another aspect, the present invention provides a method for predicting a therapeutic effect of an anti-PD-1 antibody on nasopharyngeal carcinoma, which comprises detecting a gene mutation or amplification of *CCND1*, *FGF14*, *FGF3* or *FGF4* chromosome 11q13 region in peripheral blood circulating tumour DNA and/or in a tumour tissue of an individual before treatment, wherein presence of the gene mutation or amplification of *CCND1*, *FGF14*, *FGF3* or *FGF4* chromosome 11q13 region indicates that the malignant cancer patient is not suitable for treatment with an anti-PD-1 antibody.

In another aspect, the present invention provides a method for predicting the therapeutic effect of an anti-PD-1 antibody on nasopharyngeal carcinoma, which comprises detecting the number of copies of EBV DNA in peripheral blood of an individual on day 28 of treatment, wherein there being at least 2-fold decrease in the number of copies of EBV DNA in peripheral blood indicates that the tumour patient is suitable for treatment with an anti-PD-1 antibody.

In another aspect, the present invention provides a detection kit, which contains a reagent for detecting a gene mutation or amplification of *CCND1*, *FGF14*, *FGF3* or *FGF4* chromosome 11q13 region in peripheral blood and/or in a tumour tissue of an individual.

In another aspect, the present invention provides a detection kit, which contains a reagent for detecting the number of copies of EBV DNA in peripheral blood of an individual.

In another aspect, the present invention provides a detection kit, which contains a reagent for detecting a gene mutation or amplification of *CCND1*, *FGF14*, *FGF3* or *FGF4* chromosome 11q13 region in peripheral blood and/or in a tumour tissue of an individual, and a reagent for detecting the number of copies of EBV DNA in peripheral blood of an individual.

In another aspect, the present invention provides a medical kit comprising:
one or more single drug dosage units of an anti-PD-1 antibody or an antigen-binding fragment thereof, wherein the anti-PD-1 antibody or the antigen-binding fragment thereof is as described in any of the embodiments herein; or
one or more single drug dosage units of an anti-PD-1 antibody or an antigen-binding fragment thereof, one or more single drug dosage units of gemcitabine and one or more single drug dosage units of cisplatin, wherein preferably, the anti-PD-1 antibody or the antigen-binding fragment thereof is as described in any of the embodiments herein; or
one or more single drug dosage units of the drug combination as described in any of the embodiments herein.

### Brief Description of the Drawings

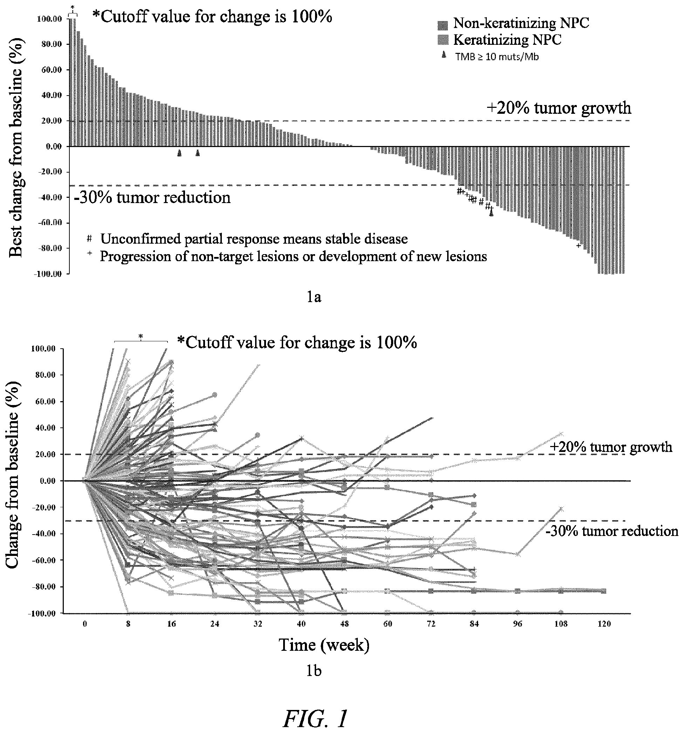

FIG. 1: results of evaluation of clinical responses according to RECIST v1.1. 1a: maximum changes from baseline in tumours evaluated for patients at baseline and at least one post-treatment imaging evaluation (n = 190), wherein the length of the bar represents the maximum decrease or minimum increase in target lesions; 1b: changes from baseline in tumour burden evaluated for individuals over time (n = 190).

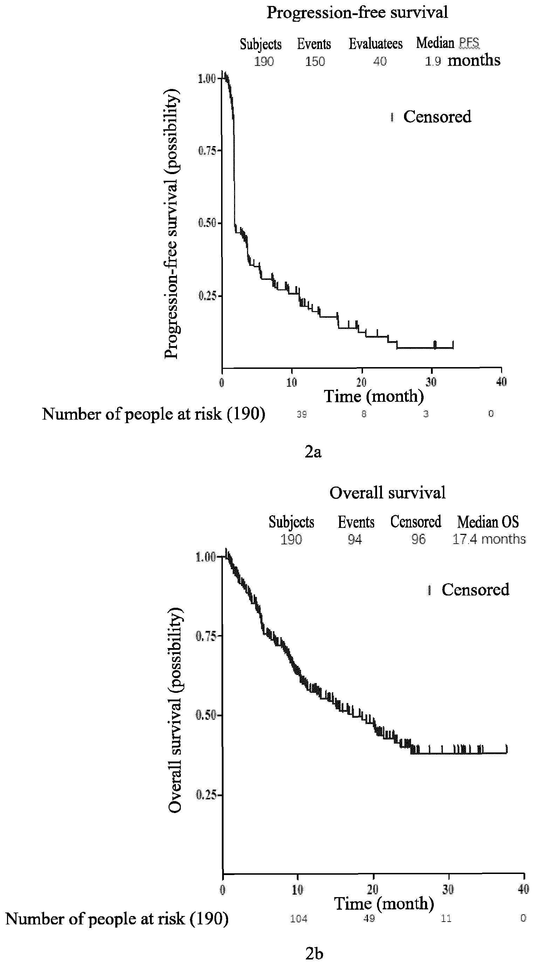

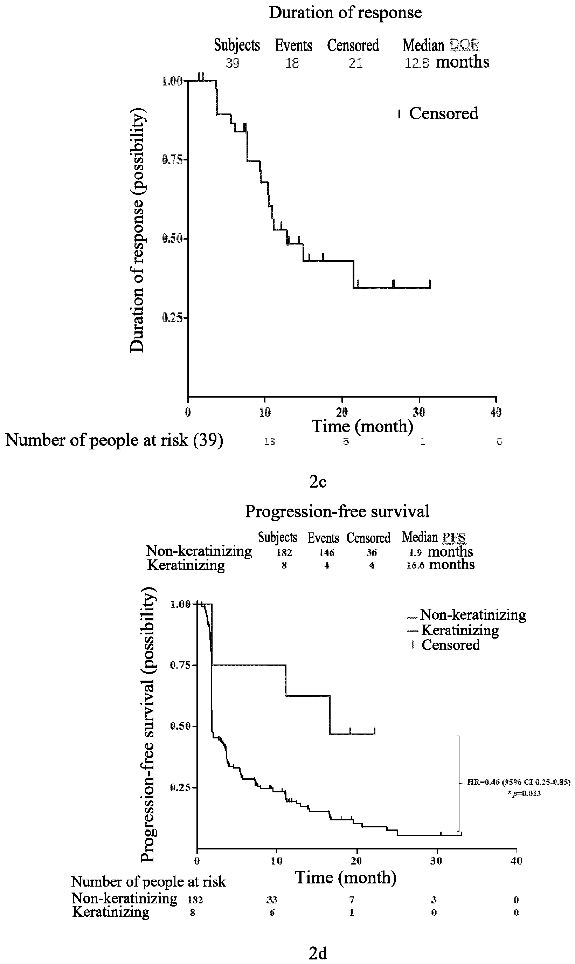

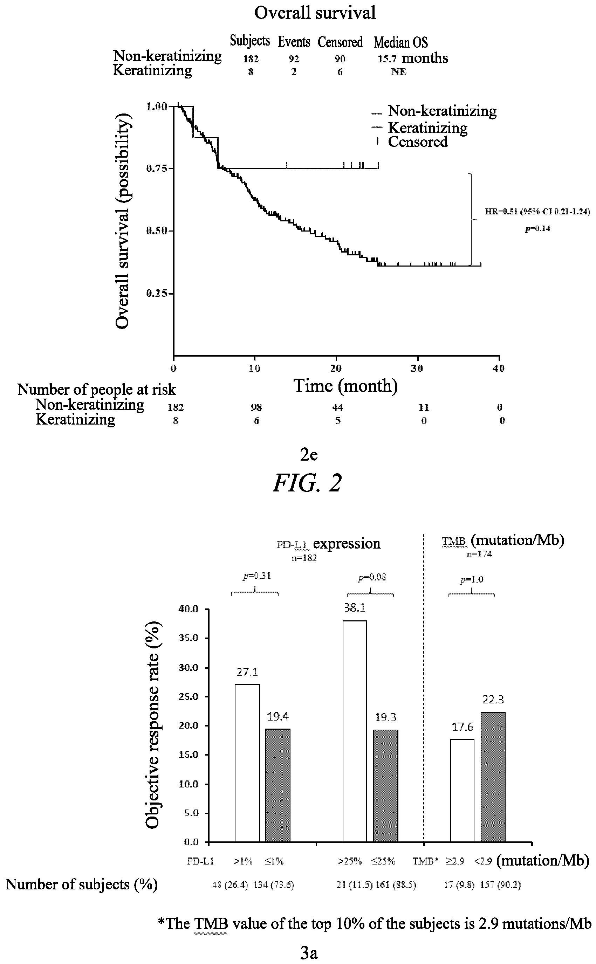

FIG. 2: 2a: progression-free survival (PFS) of nasopharyngeal carcinoma patients in this study; 2b: overall survival (OS) of nasopharyngeal carcinoma patients in this study; 2c: duration of response (DR) of nasopharyngeal carcinoma patients in this study; 2d: progression-free survival (PFS) of keratinizing and non-keratinizing nasopharyngeal carcinoma patients in this study; 2e: overall survival (OS) of keratinizing and non-keratinizing nasopharyngeal carcinoma patients in this study.

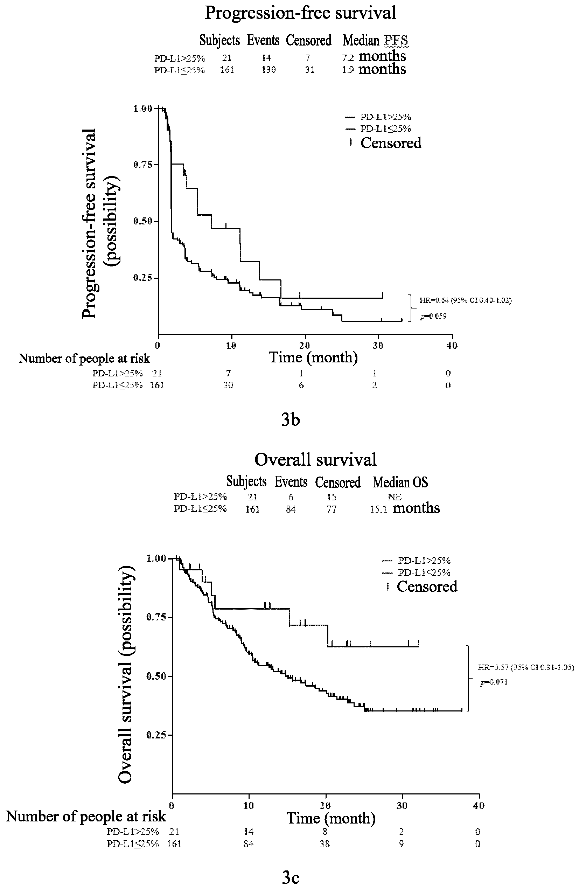

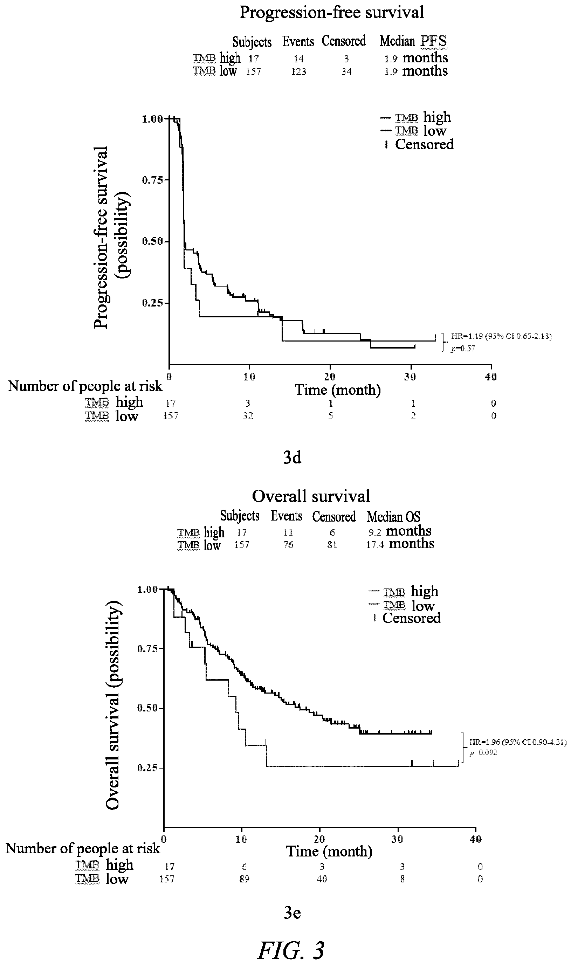

FIG. 3: 3a: the relationship between clinical response and tumour PD-L1 expression and TMB, wherein positive PD-L1 is defined as any intensity > 1% by membrane staining of tumour cells or immune cells with SP142 IHC; TMB is calculated by whole exome sequencing of somatic mutations in the coding region; 3b: progression-free survival (PFS) of patients with PD-L1+ and PD-L1-; 3c: overall survival (OS) of patients with PD-L1+ and PD-L1-; 3d: progression-free survival (PFS) of 10% patients with the highest TMB value and 90% patients with the lowest TMB value; 3e: overall survival (OS) of 10% patients with the highest TMB value and 90% patients with the lowest TMB value.

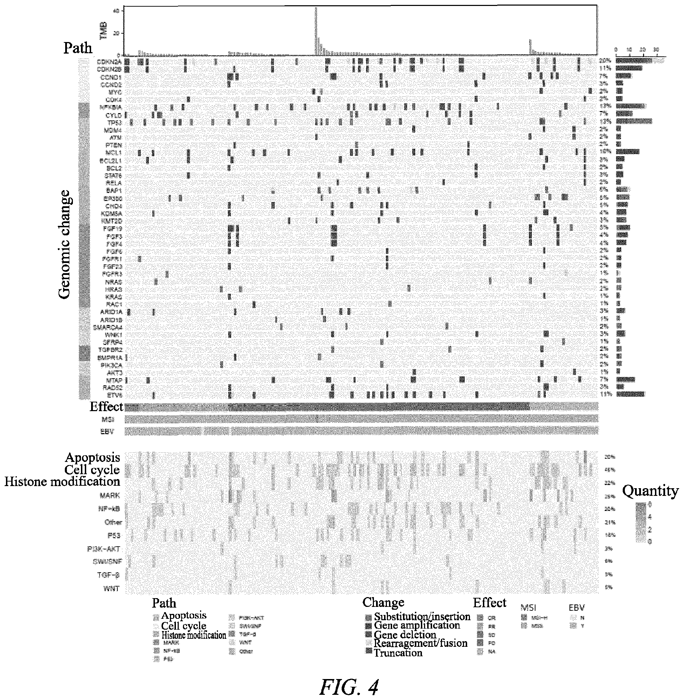

FIG. 4: the gene mutations and frequencies in 174 patients by whole exome sequencing (WES).

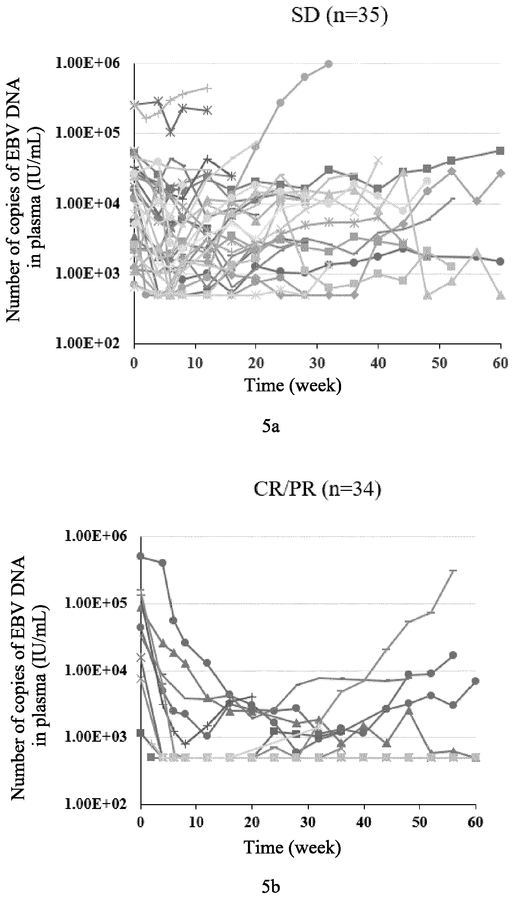

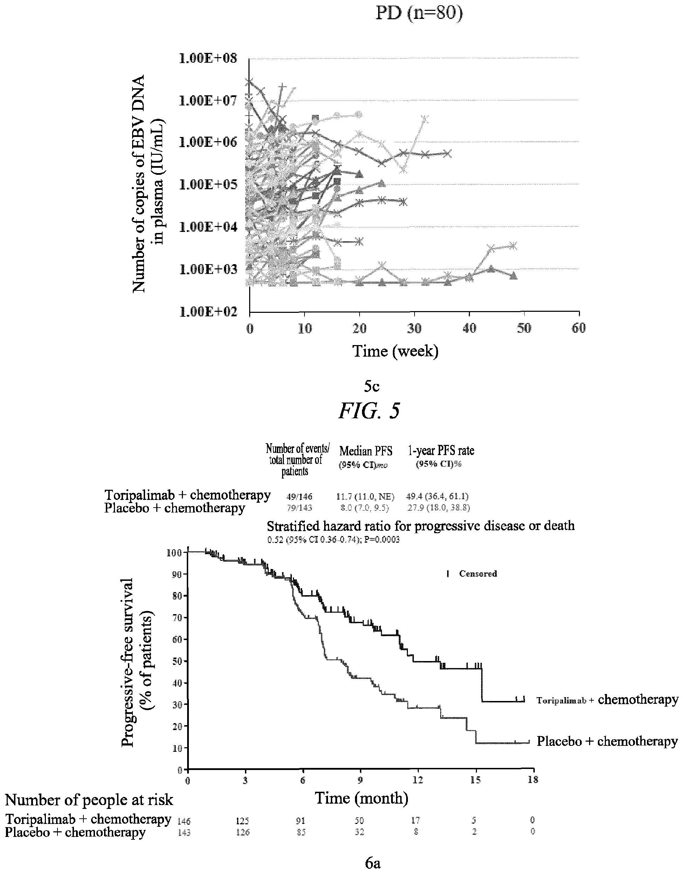

FIG. 5: 5a: the relationship between the number of copies of EBV DNA in plasma and stable disease (SD) in nasopharyngeal carcinoma patients (n = 35); 5b: the relationship between the number of copies of EBV DNA in plasma and complete response/partial response (CR/PR) of a disease in nasopharyngeal carcinoma patients (n = 34); 5c: the relationship between the number of copies of EBV DNA in plasma and progressive disease (PD) in nasopharyngeal carcinoma patients (n = 80).

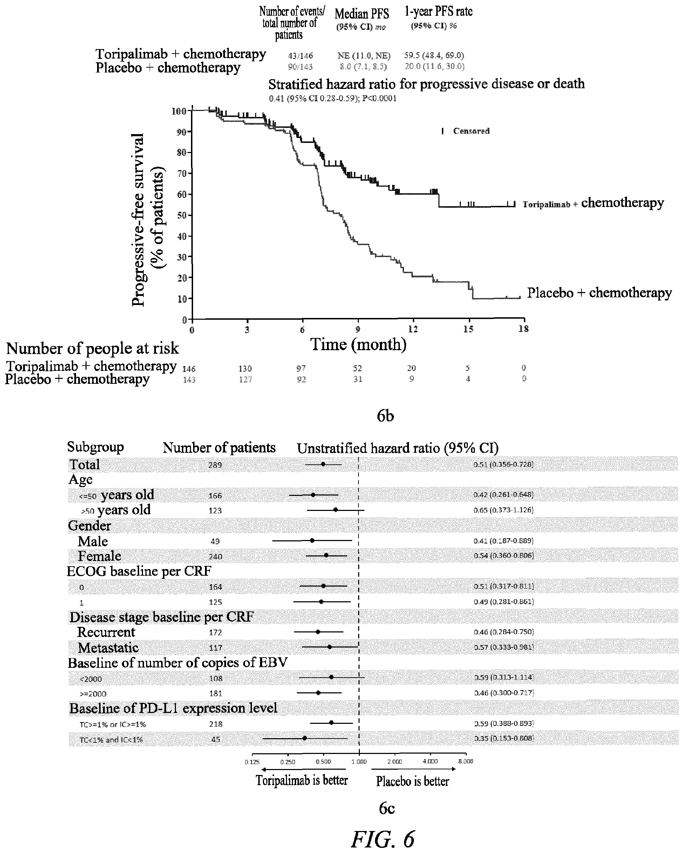

FIG. 6: 6a: PFS (intention-to-treat population) evaluated by the Independent Review Committee according to RECIST v1.1; 6b: PFS (intention-to-treat population) evaluated by the investigator according to RECIST v1.1; 6c: subgroup therapeutic effect (progression-free survival).

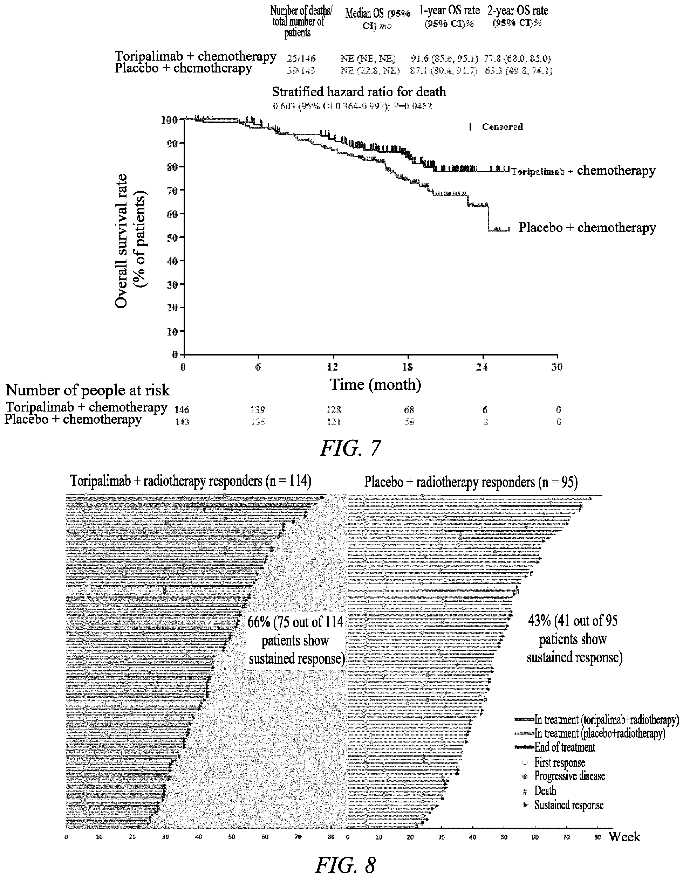

FIG. 7: overall survival (OS) (intention-to-treat population).
FIG. 8: response period evaluated by the Independent Review Committee according to RECIST v1.1.

In the figure, "chemotherapy" refers to the administration of gemcitabine and cisplatin.

### Detailed Description of Embodiments

The present invention relates to a method for treating a malignant cancer. The method of the present invention comprises administering an anti-PD-1 antibody or an antigen-binding fragment thereof to a patient in need thereof. The malignant cancer of the present invention is nasopharyngeal carcinoma. The present invention also relates to a method for using a biomarker to predict the therapeutic effect of an anti-PD-1 antibody in treatment of a malignant cancer, especially in a nasopharyngeal carcinoma patient.

### Term

In order to facilitate the understanding of the present invention, some technical and scientific terms are specifically defined below. Unless otherwise specifically indicated elsewhere herein, all technical and scientific terms used herein have the meanings as commonly understood by those of ordinary skill in the art to which the present invention belongs.

The terms "administer", "dose" and "treat" refer to the introduction of a composition containing a therapeutic agent into a subject using any of various methods or delivery systems known to those skilled in the art. The administration route of an anti-PD-1 antibody includes intravenous, intramuscular, subcutaneous, peritoneal, spinal or other parenteral administration routes, such as injection or infusion. "Parenteral administration" refers to administration routes usually by injection other than enteral or local administration, including but not limited to intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, intradural and intrasternal injection and infusion, and *in vivo* electroporation.

An "adverse event" (AE) as described herein is any adverse and often unintended or undesirable sign, symptom, or disease associated with the medical treatment practice. For example, an adverse event may be associated with the activation of the immune system or the amplification of immune system cells in response to treatment. One or more associated AEs may arise from the medical treatment, and each AE may be of the identical or different level of severity.

"Tumour burden" refers to the total amount of tumour mass distributed throughout the body. Tumour burden refers to the total number of cancer cells or the total size of a tumour throughout the body. Tumour burden can be determined by various methods known in the art, such as measuring the size of a tumour using callipers after the tumour is removed from a subject, or using imaging techniques (e.g., ultrasound, bone scanning, computed tomography (CT), or magnetic resonance imaging (MRI) scanning) when the tumour is *in vivo.*

The term "tumour size" refers to the total size of a tumour, which can be measured as the length and width of the tumour. Tumour size can be determined by various methods known in the art, such as measuring the size of a tumour using callipers after the tumour is removed from a subject, or using imaging techniques (e.g., bone scanning, ultrasound, CT, or MRI scanning) when the tumour is *in vivo.*

The terms "subject", "individual" and "object" include any organism, preferably an animal, more preferably a mammal (such as rat, mouse, dog, cat and rabbit), and most preferably a human. The terms "subject" and "patient" are used interchangeably herein.

An "antibody" as described herein refers to any form of antibody that can achieve a desired biological or binding activity. Therefore, it is used in the broadest sense, but is not limited to, monoclonal antibodies, polyclonal antibodies, multispecific antibodies, humanized full-length human antibodies, chimeric antibodies and camelid single-domain antibodies. An "antibody" specifically binds to an antigen and comprises at least two heavy (H) chains and two light (L) chains interconnected by disulphide bonds. Each heavy chain comprises a heavy chain variable region (VH) and a heavy chain constant region comprising three constant domains CH1, CH2 and CH3. Each light chain comprises a light chain variable region (VL) and a light chain constant region comprising one constant domain CL. The VH and VL regions can be further subdivided into hypervariable regions termed complementarity determining regions (CDRs), which are interspersed among more conserved regions termed framework regions (FRs). Generally, both light and heavy chain variable domains comprise FR1, CDR1, FR2, CDR2, FR3, CDR3 and FR4 from N-terminus to C-terminus. Amino acids are typically assigned to each domain according to the following definitions: Sequences of Proteins of Immunological Interest, Kabat et al.; National Institutes of Health, Bethesda, Md.; 5th edition; NIH publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917 or Chothia et al., (1989) Nature 341:878-883.

The carboxyl-terminal portion of the heavy chain can define a constant region primarily responsible for effector functions. Human light chains are generally classified as κ and λ chains. Human heavy chains are generally classified as µ, δ, γ, α and ε chains, and the isotypes of an antibody are defined as IgM, IgD, IgG, IgA and IgE, respectively. IgG subclass is well known to those skilled in the art and includes, but is not limited to, IgG1, IgG2, IgG3 and IgG4.

The term "antibody" includes: naturally occurring and non-naturally occurring Abs; monoclonal and polyclonal Abs; chimeric and humanized Abs; human or non-human Abs; fully synthetic Abs; and single chain Abs. Non-human Abs can be humanized by recombinant methods to reduce their immunogenicity in humans.

Unless otherwise specifically indicated, an "antibody fragment" or "antigen-binding fragment" as described herein refers to an antigen-binding fragment of an antibody, i.e., an antibody fragment that retains the ability of a full-length antibody to specifically bind to an antigen, e.g., a fragment that retains one or more CDR regions. Examples of an antigen-binding fragment include, but are not limited to, Fab, Fab', F(ab') 2, and Fv fragments; diabodies; linear antibodies; single chain antibody molecules; nanoantibodies and multispecific antibodies formed from antibody fragments.

A "chimeric antibody" refers to an antibody and a fragment thereof in which a portion of the heavy and/or light chain is identical or homologous to corresponding sequences of an antibody derived from a particular species (e.g., human) or belonging to a particular antibody class or subclass, while the remainder of the chain is identical or homologous to corresponding sequences of an antibody derived from another species (e.g., mouse) or belonging to another antibody class or subclass, so long as they exhibit the desired biological activity.

A "human antibody" refers to an antibody that comprises only human immunoglobulin sequences. A human antibody may contain a murine carbohydrate chain if it is produced in mice, mouse cells, or hybridomas derived from mouse cells. Similarly, a "mouse antibody" or "rat antibody" refers to an antibody that comprises only mouse or rat immunoglobulin sequences, respectively.

A "humanized antibody" refers to an antibody form containing sequences from both non-human (e.g., murine) and human antibodies. Such antibodies contain minimal sequences derived from non-human immunoglobulins. Typically, a humanized antibody will comprise substantially all of at least one and usually two variable domains, in which all or substantially all of the hypervariable loops correspond to those of a non-human immunoglobulin and all or substantially all of the FR regions are those of a human immunoglobulin. The humanized antibody optionally also comprises at least a portion of an immunoglobulin constant region (Fc), typically a human immunoglobulin constant region.

The term "nasopharyngeal carcinoma" is a malignant cancer that occurs in the nasopharyngeal cavity or the upper throat. The common clinical symptoms are nasal congestion, blood-stained nasal discharge, stuffy ears, hearing loss, diplopia, headache, etc. Epstein-Barr virus (EBV) infection is critical to the development of NPC. According to WHO classification, nasopharyngeal carcinoma has three histopathological types: keratinizing (type I), non-keratinizing (type II) and basal squamous cell carcinoma (type III). Non-keratinizing nasopharyngeal carcinoma is closely related to EBV, with higher response to radiotherapy and better overall survival.

The term "immunotherapy" refers to the treatment of a subject with a disease or at risk of infection or recurrence of disease by a method that comprises inducing, enhancing, suppressing or otherwise modifying an immune response. The "treatment" or "therapy" of a subject refers to any type of intervention or process performed on the subject, or the administration of an active agent to the subject, with the purpose of reversing, alleviating, ameliorating, slowing down or preventing the onset, progression, severity, or recurrence of symptoms, complications or conditions, or biochemical indicators associated with the disease.

"Programmed death receptor-1 (PD-1)" refers to an immunosuppressive receptor belonging to the CD28 family. PD-1 is expressed primarily on previously activated T cells *in vivo* and binds to two ligands, PD-L1 and PD-L2. The term "PD-1" as used herein includes human PD-1 (hPD-1), variants, isotypes, and species homologs of hPD-1, and analogues that share at least one common epitope with hPD-1.

A "therapeutically effective amount" or "therapeutically effective dose" of a drug or therapeutic agent is any amount of the drug that, when used alone or in combination with another therapeutic agent, protects a subject from the onset of a disease or promotes the regression of a disease as evidenced by a decrease in the severity of disease symptoms, an increase in the frequency and duration of disease symptom-free phase, or the prevention of injury or disability resulting from the affliction of the disease. The ability of a therapeutic agent to promote the regression of a disease can be assessed using various methods known to those skilled in the art, such as determining the activity of the agent in human subjects during clinical trials, in animal model systems that predict the efficacy in human, or by *in vitro* assays.

A therapeutically effective amount of a drug includes a "prophylactically effective amount" which is any amount of the drug that, when administered alone or in combination with an antineoplastic agent to a subject at risk of developing cancer or a subject having cancer recurrence, inhibits the development or recurrence of cancer.

A "biotherapeutic agent" refers to a biomolecule, such as an antibody or fusion protein, that blocks ligand/receptor signalling in any biological pathway that supports tumour maintenance and/or growth or inhibits an anti-tumour immune response.

Unless otherwise specifically indicated, "CDR" as used herein refers to a complementarity determining region of an immunoglobulin variable region defined using the Kabat numbering system.

A "therapeutic anti-PD-1 monoclonal antibody" refers to an antibody that specifically binds to the mature form of a specific PD-1 expressed on the surface of certain mammalian cells. Mature PD-1 is absent of a secretory leader sequence, or a leader peptide. The terms "PD-1" and "mature PD-1" are used interchangeably herein and should be understood as the same molecule unless otherwise specifically defined, or clearly evident from the context.

A therapeutic anti-human PD-1 antibody or anti-hPD-1 antibody as described herein refers to a monoclonal antibody that specifically binds to mature human PD-1.

A "framework region" or "FR" as described herein refers to an immunoglobulin variable region excluding CDR regions.

An "isolated antibody or antigen-binding fragment thereof' refers to a molecule that is in a purified state, and in this case, is substantially free of other biomolecules, such as nucleic acids, proteins, lipids, carbohydrates, or other materials (such as cell debris or growth medium).

A "patient", "sick person" or "subject" refers to any single subject in need of a medical method, or participating in a clinical trial and epidemiological study, or serving as a control, and is generally a mammal, including a human and other mammals, such as horses, cows, dogs or cats.

The "RECIST 1.1" as described herein refers to the definition described by Eisenhauver et al., E.A. et al., Eur. J Cancer 45:228-247 (2009) for target injury or non-target injury in the context of the measured reaction background. Before immunotherapy, it is the most commonly used standard for evaluating the response in solid tumours. However, with the advent of the era of immunity, many problems have arisen in tumour evaluation that have not appeared before. Therefore, based on the emerging phenomenon brought about by immunotherapy itself, in 2016, the RECIST working group revised the existing "RECIST v.1.1" and proposed a new criterion for judgement, namely the "irRECIST" as described herein, to better evaluate the efficacy of immunotherapy drugs.

The term "ECOG" score standard is an indicator of a patient's general health status and tolerance to treatment from the patient's physical strength. The ECOG score standard for the physical strength is as follows: 0 points, 1 point, 2 points, 3 points, 4 points and 5 points. A score of 0 means that the activity of daily living is completely normal and has no difference from that before the onset of a disease. A score of 1 means that a person is free to walk and can engage in light physical activities, including general housework or office work, but not in heavy physical activities.

A "sustained response" refers to a sustained therapeutic effect following cessation of treatment with a therapeutic agent or combination therapy as described herein. In some embodiments, the sustained response has a duration that is at least the same as the duration of treatment or at least 1.5, 2.0, 2.5 or 3 times the duration of treatment.

A "tissue section" refers to a single portion or piece of a tissue sample, such as a tissue slice cut from a sample of normal tissue or a tumour.

"Treating" cancer as described herein refers to administering a treatment regimen as described herein (e.g., administering an anti-PD-1 antibody) to a subject with or diagnosed with cancer to achieve at least one positive therapeutic effect (e.g., a decrease in the number of cancer cells, a decrease in tumour volume, a reduction in the rate at which cancer cells infiltrate into peripheral organs, or a reduction in the rate at which tumours metastasize or grow). Positive therapeutic effects in cancer can be measured in various ways (see W. A. Weber, J. Nucl. Med., 50:1S-10S (2009)). For example, T/C ≦ 42% for tumour growth inhibition is the minimum level of anti-tumour activity according to the NCI criteria. T/C (%) = median treated tumour volume/median control tumour volume × 100. PFS (also called "time to tumour progression") refers to the length of time the cancer does not grow during and after treatment, and includes the amount of time a patient experiences CR or PR and the amount of time a patient experiences SD. DFS refers to the length of time a patient remains disease-free during and after treatment. OS refers to an extension of life expectancy compared with an initial or untreated individual or patient. The treatment regimen of the combination of the present invention effective in treating a cancer patient may vary depending upon a variety of factors such as the disease state, age and weight of the patient, and the ability of the therapy to elicit an anti-cancer response in the subject. Embodiments of the present invention may not achieve an effective positive therapeutic effect in every subject, but should be effective and achieve a positive therapeutic effect in a statistically significant number of subjects.

The terms "mode of administration" and "administration regimen" are used interchangeably and refer to the dosage and time of use of each therapeutic agent in the combination of the present invention.

The term "immunohistochemistry (IHC)" refers to a method for determining antigens (polypeptides and proteins) in tissue cells by developing chromogenic agents (fluoresceins, enzymes, metal ions and isotopes) that label antibodies by means of chemical reaction based on the principle that antigens specifically bind to antibodies, and performing localized, qualitative and relatively quantitative studies on those antigens. In some embodiments of the present invention, prior to treatment with an anti-PD-1 antibody, a tumour tissue sample from a subject is tested for PD-L1 by means of a staining experiment with an anti-human PD-L1 antibody SP142 from Roche (Cat No: M4422). In some embodiments, intensity >_ 1% by membrane staining of tumour cells is defined as positive PD-L1.

Herein, the term "cancer" or "malignant cancer" refers to a wide range of diseases characterized by uncontrolled growth of abnormal cells in the body. Unregulated cell division, growth division and growth lead to the formation of malignant cancers, which invade adjacent tissues and can also metastasize to the distal parts of the body through the lymphatic system or bloodstream. Examples of cancers suitable for treatment or prevention by the method, drug, and kit of the present invention include, but are not limited to, carcinoma, lymphoma, leukaemia, blastoma, and sarcoma. More specific examples of cancers include squamous cell carcinoma, myeloma, small cell lung cancer, non-small cell lung cancer, glioma, Hodgkin's lymphoma, non-Hodgkin's lymphoma, acute myeloid leukaemia, multiple myeloma, gastrointestinal cancer, renal cancer, ovarian cancer, liver cancer, lymphoblastic leukaemia, lymphocytic leukaemia, colorectal cancer, endometrial cancer, kidney cancer, prostate cancer, thyroid cancer, melanoma, chondrosarcoma, neuroblastoma, pancreatic cancer, glioblastoma multiforme, nasopharyngeal carcinoma, cervical cancer, brain cancer, gastric cancer, bladder cancer, hepatocellular carcinoma, breast cancer, colon cancer and head and neck cancer.

Herein, the term "tumour mutation burden (TMB)" refers to the total number of gene coding errors, base substitutions, and gene insertion or deletion errors detected in somatic cells per million bases. In some embodiments of the present invention, tumour mutation burden (TMB) is estimated by analysis of somatic mutations, including coding base substitutions and megabase insertions of the panel sequences studied.

In the following paragraphs, various aspects of the present invention are further described in detail.

### Anti-PD-1 antibody

Herein, an "anti-PD-1 antibody" refers to any chemical compound or biomolecule that binds to a PD-1 receptor, blocks the binding of PD-L1 expressed on cancer cells to PD-1 expressed on immune cells (T, B and NK cells), and preferably blocks the binding of PD-L2 expressed on cancer cells to PD-1 expressed on immune cells. Alternative nouns or synonyms for PD-1 and its ligands include: PDCD1, PD1, CD279 and SLEB2 forPD-1; PDCD1L1, PDL1, B7-H1, B7H1, B7-4, CD274 and B7-H for PD-L1; and PDCD1L2, PDL2, B7-DC and CD273 for PD-L2. In any of the treatment methods, drugs and uses of the present invention for treating a human individual, the anti-PD-1 antibody blocks the binding of human PD-L1 to human PD-1, and preferably blocks the binding of both human PD-L1 and PD-L2 to human PD1. The amino acid sequence of human PD-1 can be found at NCBI locus number: NP_005009. The amino acid sequences of human PD-L1 and PD-L2 can be found at NCBI locus numbers: NP_054862 and NP_079515, respectively.

Unless otherwise indicated or described, the term "anti-PD-1 antibody" when referred to herein includes an antigen-binding fragment thereof.

The anti-PD-1 antibody suitable for use in any of the uses, therapies, drugs and kits of the present invention binds to PD-1 with high specificity and high affinity, blocks the binding of PD-L1/2 to PD-1, and inhibits PD-1 signal transduction, thereby achieving an immunosuppressive effect. In any of the uses, therapies, drugs and kits disclosed herein, the anti-PD-1 antibody includes a full-length antibody itself, as well as an antigen-binding portion or fragment that binds to a PD-1 receptor and exhibits functional properties similar to an intact Ab in inhibiting ligand binding and upregulating the immune system. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized or human Ab or an antigen-binding fragment thereof. In certain embodiments for treating a human individual, the Ab is a humanized Ab.

In some embodiments, the anti-PD-1 antibody for use in any of the uses, therapies, drugs and kits of the present invention includes a monoclonal antibody (mAb) or an antigen-binding fragment thereof that specifically binds to PD-1, and preferably specifically binds to human PD-1. The mAb may be a human antibody, a humanized antibody, or a chimeric antibody, and may include a human constant region. In some embodiments, the constant region is selected from the group consisting of human IgG1, IgG2, IgG3 and IgG4 constant regions; preferably, the anti-PD-1 antibody or the antigen-binding fragment thereof suitable for use in any of the uses, therapies, drugs and kits of the present invention comprises a heavy chain constant region of human IgG1 or IgG4 isotype, more preferably a human IgG4 constant region. In some embodiments, the sequence of the IgG4 heavy chain constant region of the anti-PD-1 antibody or the antigen-binding fragment thereof comprises the S228P mutation that replaces a serine residue in the hinge region with a proline residue typically present at the corresponding position of an antibody of IgG1 isotype.

Preferably, in any of the embodiments of the uses, therapies, drugs and kits of the present invention, the anti-PD-1 antibody is a monoclonal antibody or an antigen-binding fragment thereof, wherein the light chain CDRs comprise amino acid sequences as set forth in SEQ ID NOs: 1, 2 and 3, and the heavy chain CDRs comprise amino acid sequences as set forth in SEQ ID NOs: 4, 5 and 6.

More preferably, in any of the embodiments of the uses, therapies, drugs and kits of the present invention, the anti-PD-1 antibody is a monoclonal antibody that specifically binds to human PD-1 and comprises: (a) a light chain variable region comprising SEQ ID NO: 7, and (b) a heavy chain variable region comprising SEQ ID NO: 8.

Further preferably, in any of the embodiments of the uses, therapies, drugs and kits of the present invention, the anti-PD-1 antibody is a monoclonal antibody that specifically binds to human PD-1 and comprises: (a) a light chain comprising SEQ ID NO: 9, and (b) a heavy chain comprising SEQ ID NO: 10.

Table A below provides the amino acid sequence numbering of the light chain CDRs and heavy chain CDRs of an exemplary anti-PD-1 antibody mAb for use in the uses, therapies, drugs and kits of the present invention.

**Table A: Light and heavy chain CDRs of an exemplary anti-human PD-1 antibody**

| | |
|---|---|
| LCDR1 | SEQ ID NO: 1 |
| LCDR2 | SEQ ID NO: 2 |
| LCDR3 | SEQ ID NO: 3 |
| HCDR1 | SEQ ID NO: 4 |
| HCDR2 | SEQ ID NO: 5 |
| HCDR3 | SEQ ID NO: 6 |

Examples of anti-PD-1 antibodies that bind to human PD-1 and can be used in the uses, therapies, drugs and kits of the present invention are described in WO 2014206107. Human PD-1 mAbs that can be used as anti-PD-1 antibodies in the uses, therapies, drugs and kits of the present invention include any of the anti-PD-1 antibodies as described in WO 2014206107, including toripalimab (a humanized IgG4 mAb having the structure as described in WHO Drug Information, vol. 32, no. 2, pp. 372-373 (2018) and comprising light and heavy chain amino acid sequences as set forth in SEQ ID NOs: 9 and 10). In a preferred embodiment, the anti-PD-1 antibody that can be used in any of the uses, therapies, drugs and kits of the present invention is selected from humanized antibodies 38, 39, 41 and 48 as described in WO 2014206107. In a particularly preferred embodiment, the anti-PD-1 antibody that can be used in any of the uses, therapies, drugs and kits of the present invention is toripalimab.

The anti-PD-1 antibody that can be used in any of the uses, therapies, drugs and kits of the present invention also includes nivolumab and pembrolizumab approved by FDA.

In certain embodiments, the anti-PD-1 antibody that can be used in any of the uses, therapies, drugs and kits of the present invention also includes anti-PD-L1 monoclonal antibodies that specifically bind to PD-L1 to block the binding of PD-L1 to PD-1, such as nivolumab, pembrolizumab, toripalimab, sintilimab, camrelizumab, tislelizumab, and cemiplimab.

"PD-L1" expression or "PD-L2" expression as described herein refers to any detectable expression level of a specific PD-L protein on the surface of a cell or a specific PD-L mRNA within a cell or tissue. PD-L protein expression can be detected in IHC analysis of a cancer tissue section or by flow cytometry using a diagnostic PD-L antibody. Alternatively, PD-L protein expression of tumour cells can be detected by PET imaging using a binding agent that specifically binds to a desired PD-L target (such as PD-L1 or PD-L2).

Methods for quantifying PD-L1 protein expression in IHC analysis of a cancer tissue section are found in, but are not limited to, Thompson, R. H. et al., PNAS 101(49): 17174-17179 (2004); Taube, J. M. et al., Sci Transl Med 4, 127ra37 (2012); and Toplian, S. L. et al., New Eng. J. Med. 366(26): 2443-2454 (2012), and the like.

One method employs a simple binary endpoint of positive or negative PD-L 1 expression, where a positive result is defined by the percentage of tumour cells showing histological evidence of cell surface membrane staining. The case where tumour cells on a cancer tissue section account for more than 1% of the total tumour cells is defined as positive PD-L1 expression.

In another method, PD-L1 expression in a cancer tissue section is quantified in tumour cells as well as in infiltrating immune cells. The percentages of tumour cells and infiltrating immune cells exhibiting membrane staining are quantified individually as ≤ 1%, 1% to 50%, and subsequent 50% up to 100%. For tumour cells, the PD-L1 expression is counted as negative if the score is ≤ 1%, and counted as positive if the score is > 1%.

In some embodiments, the expression level of PD-L1 by malignant cells and/or by infiltrating immune cells within the tumour is determined to be "overexpressed" or "elevated" based on comparison with the expression level of PD-L1 by an appropriate control. For example, the protein or mRNA expression level of control PD-L1 can be a quantified level in non-malignant cells of the same type or in sections from matched normal tissues.

### Gemcitabine

Gemcitabine is a new cytosine nucleoside derivative with the structure as shown in the following formula:

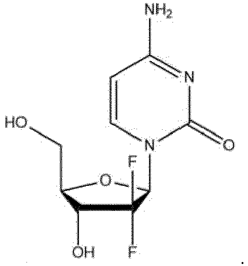

Like cytarabine, gemcitabine is activated by a deoxycytosine kinase and metabolized by a cytosine nucleoside deaminase after entering the human body. The main metabolite of gemcitabine, difluorodeoxycytidine, is incorporated into DNA in cells and mainly acts on G1/S stage. However, the difference is that in addition to incorporation into DNA, difluorodeoxycytidine can inhibit a nucleotide reductase, resulting in a decrease in intracellular deoxynucleoside triphosphate. Another difference from cytarabine is that gemcitabine can inhibit a deoxycytosine deaminase to reduce the degradation of intracellular metabolites, which has a self synergistic effect.

In some embodiments of the present invention, gemcitabine may also refer to a composition, which comprises a therapeutically effective amount of the compound as shown in the above structural formula, a pharmaceutically acceptable salt thereof, and a pharmaceutically acceptable excipient.

### Cisplatin

Cisplatin is a platinum-containing anti-cancer drug, namely cis-dichlorodiammine platinum, which is orange yellow or yellow crystalline powder, slightly soluble in water and easily soluble in dimethylformamide, and can be gradually converted into a trans form and hydrolysed in an aqueous solution. Cisplatin is a compound with the structure as shown in the following formula:

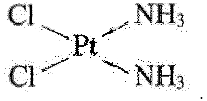

In some embodiments of the present invention, cisplatin may also refer to a composition, which comprises a therapeutically effective amount of the compound as shown in the above formula, a pharmaceutically acceptable salt thereof, and a pharmaceutically acceptable excipient.

### Drug combination

The present invention also provides a drug combination, which contains the anti-PD-1 antibody as described herein, gemcitabine and cisplatin. In the drug combination, the anti-PD-1 antibody, gemcitabine and cisplatin can be provided in the form of a mixture of the three (that is, in the form of a pharmaceutical composition), or in the form of a mixture of any two and another independent formulation, or in the form of an independent formulation of each. In some embodiments, the drug combination contains a three-week administration dose, including one dose of the anti-PD-1 antibody as described herein, two doses of gemcitabine, and one dose of cisplatin. When present in the form of an independent formulation, each formulation contains a pharmaceutically acceptable carrier in addition to the active ingredient.

In some embodiments, the anti-PD-1 antibody of the present invention may be as described in any of the embodiments herein, more preferably an antibody comprising light chain CDRs with amino acid sequences as set forth in SEQ ID NOs: 1, 2 and 3 and heavy chain CDRs with amino acid sequences as set forth in SEQ ID NOs: 4, 5 and 6, more preferably a monoclonal antibody comprising a light chain variable region with an amino acid sequence as set forth in SEQ ID NO: 7 and a heavy chain variable region with an amino acid sequence as set forth in SEQ ID NO: 8, more preferably a monoclonal antibody comprising a light chain with an amino acid sequence as set forth in SEQ ID NO: 9 and a heavy chain with an amino acid sequence as set forth in SEQ ID NO: 10, more preferably the humanized antibodies 38, 39, 41 and 48 as described in WO 2014206107, and most preferably toripalimab.

The drug combination of the present invention may also comprise one or more additional therapeutic agents. The additional therapeutic agents may be, for example, chemotherapeutic agents, biotherapeutic agents, and immunogenic agents (for example, attenuated cancer cells, tumour antigens, antigen presenting cells (such as dendritic cells pulsed with tumour-derived antigens or nucleic acids), immunostimulating cytokines (such as IL-2, IFN tumour, and GM-CSF), and cells transfected with genes encoding the immunostimulating cytokines (such as, but not limited to, GM-CSF)).

### Dosage and administration regimen

The anti-PD-1 antibody of the present invention may be administered by continuous infusion or by interval doses. The single administration dose may range from about 0.01 mg/kg body weight to about 20 mg/kg body weight and about 0.1 mg/kg body weight to about 10 mg/kg body weight of an individual, or may be a fixed dose of about 120 mg to about 480 mg. For example, the dose may be about 0.1 mg/kg body weight, about 0.3 mg/kg body weight, about 1 mg/kg body weight, about 2 mg/kg body weight, about 3 mg/kg body weight, about 4 mg/kg body weight, about 5 mg/kg body weight, about 6 mg/kg body weight, about 7 mg/kg body weight, about 8 mg/kg body weight, about 9 mg/kg body weight or about 10 mg/kg body weight of an individual, or a fixed dose of about 120 mg, 240 mg, 360 mg or 480 mg. An administration regimen is usually designed to achieve such exposure which results in sustained receptor occupancy (RO) based on the typical pharmacokinetic properties of Abs. A representative administration regimen may be about once a week, about once every two weeks, about once every three weeks, about once every four weeks, about once a month, or longer. In some embodiments, the anti-PD-1 antibody is administered to an individual about once every three weeks. In some embodiments, the anti-PD-1 antibody is administered to an individual about once every two weeks.

In some embodiments, the anti-PD-1 antibody of the present invention is toripalimab which is administered at a single dose selected from about 1 mg/kg body weight to about 5 mg/kg body weight of an individual. In some embodiments, toripalimab is administered intravenously at a single dose selected from a dose of about 1 mg/kg body weight, 2 mg/kg body weight, 3 mg/kg body weight, 4 mg/kg body weight and 5 mg/kg body weight, or at a fixed dose of 120 mg, 240 mg and 360 mg. In some preferred embodiments, toripalimab is administered as a liquid drug, and the selected dose of the drug is administered by intravenous infusion over a period of 30 minutes to 60 minutes. In some embodiments, toripalimab is administered at a dose of about 3 mg/kg or at a fixed dose of about 240 mg once every three weeks (Q3W) by intravenous infusion over a 30-minute period. In some embodiments, toripalimab is administered at a dose of about 3 mg/kg or at a fixed dose of about 240 mg once every two weeks (Q2W) by intravenous infusion over a 30-minute period.

Each therapeutic agent in the drug combination of the present invention may be administered simultaneously (i.e., in the same pharmaceutical composition), concurrently (i.e., administered as a separate pharmaceutical formulation one after another in any order), or sequentially in any order. Sequential administration is particularly useful when the therapeutic agents in the drug combination can be administered in different dosage forms (one drug is a tablet or capsule and the other is a sterile liquid formulation) and/or in different administration schedules (e.g., a chemotherapeutic agent is administered at least daily and a biotherapeutic agent is administered less frequently (e.g., once a week, once every two weeks or once every three weeks).

In some embodiments, at least one therapeutic agent in the drug combination is administered using the same dosage regimen (therapeutic dose, frequency, and duration) that is commonly used when the agent is used to treat the same tumour in a single therapy. In other embodiments, the patient receives at least one therapeutic agent in the combination therapy in a smaller total amount, such as at a smaller dose, at a less frequent dose, and/or in a shorter duration of treatment, than when the agent is used in a single therapy.

Each therapeutic agent in the drug combination of the present invention may be administered orally or parenterally, including intravenous, intramuscular, intraperitoneal, subcutaneous, rectal, local and transdermal routes.

The gemcitabine of the present invention is administered at the approved or recommended dose for continuous treatment until clinical effects are observed or until unacceptable toxicity or progressive disease occurs. In some embodiments, the gemcitabine of the present invention is administered at a single dose selected from about 600 mg/m² body surface area to about 1400 mg/m² body surface area. In some embodiments, gemcitabine is administered at a single dose selected from any dose of about 800 mg/m² body surface area, 900 mg/m² body surface area, 1000 mg/m² body surface area, 1100 mg/m² body surface area, and 1200 mg/m² body surface area. A representative administration regimen may be about once a week, once every two weeks, once every three weeks, twice every three weeks, once every four weeks or once a month. In some embodiments, gemcitabine is administered to an individual twice every three weeks. In some embodiments, gemcitabine is administered on day 1 and day 8 of each treatment cycle, respectively. In some embodiments, gemcitabine is administered twice every three weeks at a dose of about 1000 mg/m² body surface area.

The cisplatin of the present invention is administered at the approved or recommended dose for continuous treatment until entering the disease maintenance stage or until unacceptable toxicity or progressive disease occurs. In some embodiments, the cisplatin of the present invention is administered at a single dose selected from about 40 mg/m² body surface area to about 120 mg/m² body surface area. In some embodiments, cisplatin is administered at a single dose selected from any dose of about 60 mg/m² body surface area, 70 mg/m² body surface area, 80 mg/m² body surface area, 90 mg/m² body surface area, and 100 mg/m² body surface area. A representative administration regimen may be about once a week, once every two weeks, once every three weeks, once every four weeks or once a month. In some embodiments, cisplatin is administered to an individual once every three weeks. In some embodiments, cisplatin is administered once every three weeks at a dose of about 80 mg/m² body surface area.

In some embodiments, toripalimab is administered Q3W at a fixed dose of about 240 mg; gemcitabine is administered twice every three weeks at a dose of about 1000 mg/m² body surface area; and cisplatin is administered Q3W at a dose of about 80 mg/m² body surface area.

In some embodiments, on the day of administration of toripalimab, gemcitabine may be administered before or after the administration of toripalimab, and cisplatin may be administered before or after the administration of toripalimab.

The administration periods of the anti-PD-1 antibody of the present invention, gemcitabine and cisplatin may be the identical or different, including one week, two weeks, three weeks, one month, two months, three months, four months, five months, half a year or longer; and optionally, each administration period lasts for the identical or different period of time and is at identical or different intervals. For example, in some embodiments, toripalimab is administered once every three weeks at a fixed dose of about 240 mg; gemcitabine is administered twice every three weeks at a dose of about 1000 mg/m² body surface area; and cisplatin is administered once every three weeks at a dose of about 80 mg/m² body surface area, wherein the administration period of the three drugs is three weeks.

### Treatment method and use

The present invention provides the use of the anti-PD-1 antibody or the antigen-binding fragment thereof of the present invention and optionally gemcitabine and cisplatin in preparing a drug for preventing or treating a malignant cancer.

The present invention provides a method for preventing or treating a malignant cancer, which comprises administering an effective amount of the anti-PD-1 antibody or the antigen-binding fragment thereof of the present invention, and optionally gemcitabine and cisplatin to an individual in need thereof. The effective amount includes a prophylactically effective amount and a therapeutically effective amount. In a preferred embodiment, the administration regimen used in the prevention or treatment method (including dose, administration frequency, administration order, etc.) is as described in any of the embodiments above.

The present invention provides the anti-PD-1 antibody or the antigen-binding fragment thereof of the present invention, for use in preventing or treating a malignant cancer. The present invention also provides a drug combination of the anti-PD-1 antibody or the antigen-binding fragment thereof of the present invention and gemcitabine and cisplatin, for use in preventing or treating a malignant cancer.

The malignant cancer of the present invention can be as described in any of the preceding embodiments. Preferably, the malignant cancer of the present invention is nasopharyngeal carcinoma, and preferably, the malignant cancer of the present invention is recurrent or metastatic nasopharyngeal carcinoma.

Preferably, the method, use, anti-PD-1 antibody and drug combination as described in any of the embodiments of the present invention are particularly suitable for keratinizing nasopharyngeal carcinoma and non-keratinizing nasopharyngeal carcinoma, preferably keratinizing nasopharyngeal carcinoma.

Preferably, the method, use, anti-PD-1 antibody and drug combination as described in any of the embodiments of the present invention are particularly suitable for a malignant cancer with positive PD-L1 expression in a cancer tissue section by immunohistochemical staining analysis, preferably a malignant cancer with PD-L1 expression > 25% in a cancer tissue section by immunohistochemical staining analysis.

Preferably, the method, use, anti-PD-1 antibody and drug combination as described in any of the embodiments of the present invention are particularly suitable for a malignant cancer in which no gene amplification of *CCND1*, *FGF14*, *FGF3* or *FGF4* chromosome 11q13 region is detected in peripheral blood circulating tumour DNA or in a tumour tissue.

Preferably, the method, use, anti-PD-1 antibody and drug combination as described in any of the embodiments of the present invention are particularly suitable for a malignant cancer in which there is at least 2-fold decrease in the number of copies of EBV DNA in peripheral blood (on day 28 of treatment).

The preferred anti-PD-1 antibody for a malignant cancer may be as described in any of the embodiments herein, more preferably an antibody comprising light chain CDRs with amino acid sequences as set forth in SEQ ID NOs: 1, 2 and 3 and heavy chain CDRs with amino acid sequences as set forth in SEQ ID NOs: 4, 5 and 6, more preferably a monoclonal antibody comprising a light chain variable region with an amino acid sequence as set forth in SEQ ID NO: 7 and a heavy chain variable region with an amino acid sequence as set forth in SEQ ID NO: 8, more preferably a monoclonal antibody comprising a light chain with an amino acid sequence as set forth in SEQ ID NO: 9 and a heavy chain with an amino acid sequence as set forth in SEQ ID NO: 10, more preferably the humanized antibodies 38, 39, 41 and 48 as described in WO 2014206107, and most preferably toripalimab.

In a particularly preferred embodiment, the present invention provides a method for preventing or treating nasopharyngeal carcinoma, the method comprising administering a therapeutically effective amount of toripalimab or a drug combination as described herein to a nasopharyngeal carcinoma patient. Preferably, the patient has positive PD-L1 expression. In certain embodiments, the preferred nasopharyngeal carcinoma is keratinizing nasopharyngeal carcinoma. In certain embodiments, the preferred nasopharyngeal carcinoma patient is a patient with nasopharyngeal carcinoma in which no gene amplification of *CCND1*, *FGF14*, *FGF3* or *FGF4* chromosome 11q13 region is detected in peripheral blood circulating tumour DNA or in a tumour tissue. In certain embodiments, the patient is preferably a nasopharyngeal carcinoma patient with at least 2-fold decrease in the number of copies of EBV DNA in peripheral blood on day 28 of treatment.

In a particularly preferred embodiment, the present invention provides the use of an anti-PD-1 antibody or an antigen-binding fragment thereof or a drug combination as described herein in preparing a drug for preventing or treating nasopharyngeal carcinoma. Preferably, the nasopharyngeal carcinoma has positive PD-L1 expression in a cancer tissue section by immunohistochemical staining analysis. In certain embodiments, the preferred nasopharyngeal carcinoma is a nasopharyngeal carcinoma in which no gene amplification of *CCND1*, *FGF14*, *FGF3* or *FGF4* chromosome 11q13 region is detected in peripheral blood circulating tumour DNA or in a tumour tissue. In certain embodiments, the nasopharyngeal carcinoma is preferably a nasopharyngeal carcinoma in which there is at least 2-fold decrease in the number of copies of EBV DNA in peripheral blood on day 28 of treatment.

The therapeutic agent of the present invention can constitute a pharmaceutical composition, such as a pharmaceutical composition comprising the anti-PD-1 antibody of the present invention or/and other anti-cancer agents other than the anti-PD-1 antibody, and other pharmaceutically acceptable carriers. As described in the present invention, a "pharmaceutically acceptable carrier" includes any and all solvents, dispersion media, coatings, antibacterial and antifungal agents, isotonic agents and absorption delaying agents, and the like that are physiologically compatible. Preferably, the carrier suitable for use in a composition containing an anti-PD-1 antibody is suitable for intravenous, intramuscular, subcutaneous, parenteral, spinal, or epidermal administration, such as by injection or infusion, while the carrier for use in a composition containing other anti-cancer agents is suitable for parenteral administration, such as oral administration. The pharmaceutical composition of the present invention can contain one or more pharmaceutically acceptable salts, antioxidants, water, non-aqueous carriers, and/or adjuvants such as preserving agents, wetting agents, emulsifying agents, and dispersing agents. In a preferred embodiment, the anti-cancer agents other than the anti-PD-1 antibody include gemcitabine and cisplatin.

The administration regimen is adjusted to provide the best desired response, such as the maximum treatment response and/or the minimum adverse effect. For the administration of an anti-PD-1 antibody (including when combined with another anti-cancer agent), the dose may range from about 0.01 mg/kg body weight to about 20 mg/kg body weight and about 0.1 mg/kg body weight to about 10 mg/kg body weight of an individual, or may be a fixed dose of 120 mg, 240 mg, 360 mg or 480 mg. For example, the dose may be about 0.1 mg/kg body weight, about 0.3 mg/kg body weight, about 1 mg/kg body weight, about 2 mg/kg body weight, about 3 mg/kg body weight, about 5 mg/kg body weight, or about 10 mg/kg body weight of an individual. An administration regimen is usually designed to achieve such exposure which results in sustained receptor occupancy (RO) based on the typical pharmacokinetic properties of Abs. A representative administration regimen may be about once a week, about once every two weeks, about once every three weeks, about once every four weeks, about once a month, or longer. In some embodiments, the anti-PD-1 antibody is administered to an individual about once every two weeks.

### Method for predicting therapeutic effect of anti-PD-1 antibody on malignant cancer

Herein, the term "gene amplification" refers to a process in which the copy number of a gene encoding a specific protein is increased selectively while other genes are not increased proportionally. Under natural conditions, the gene amplification is achieved by excision of repeats of a gene from the chromosome followed by extrachromosomal replication in a plasmid, or by generation of RNA transcripts from the entire repeats of ribosomal RNA followed by transcription to generate additional copies of the original DNA molecule. In some embodiments of the present invention, gene sequencing analysis is disclosed.

In some embodiments of the present invention, the subjects of the present invention have some unique gene amplifications, for example, some subjects have the gene amplification of *CCND1*, *FGF14*, *FGF3* or *FGF4* chromosome 11q13 region. In some embodiments of the present invention, the patient has the gene amplification of *CCND1*, *FGF14*, *FGF3* or *FGF4* chromosome 11q13 region, indicating that the therapeutic effect of treating the patient with the anti-PD-1 antibody of the present invention alone is not ideal.

In some embodiments of the present invention, the number of copies of EBV DNA in peripheral blood on day 28 of treatment is reduced in some subjects of the present invention. In some embodiments of the present invention, there being at least 2-fold decrease in the number of copies of EBV DNA in peripheral blood indicates that the therapeutic effect of treating the patient with the anti-PD-1 antibody of the present invention is better.

Therefore, the present invention provides a method for predicting the therapeutic effect of the anti-PD-1 antibody of the present invention, especially toripalimab, on a malignant cancer in an individual, comprising detecting a biomarker in patients' peripheral blood before treatment, wherein the biomarker is selected from but not limited to *CCND1*, *FGF14*, *FGF3* or *FGF4* chromosome 11q13 region mutations, or detecting the number of copies of EBV DNA in patients' peripheral blood on day 28 of treatment.

The present invention also includes a method for predicting the therapeutic effect of an anti-PD-1 antibody in a tumour patient by detecting presence of the gene amplification of *CCND1*, *FGF14*, *FGF3* or *FGF4* chromosome 11q13 region. Preferably, presence of the gene amplification of *CCND1*, *FGF14*, *FGF3* or *FGF4* chromosome 11q13 region indicates that the tumour patient is not suitable for treatment with the anti-PD-1 antibody alone. Preferably, the tumour patient is selected from a nasopharyngeal carcinoma patient.

The present invention also includes a method for predicting the therapeutic effect of an anti-PD-1 antibody in a tumour patient by detecting the number of copies of EBV DNA in peripheral blood of the tumour patient on day 28 of administration. Preferably, there being at least 2-fold decrease in the number of copies of EBV DNA in peripheral blood (the number of copies of EBV DNA in peripheral blood before administration on day 0/the number of copies of EBV DNA in peripheral blood on day 28 ≥ 2) indicates that the tumour patient is suitable for treatment with the anti-PD-1 antibody. Preferably, the tumour patient is selected from a nasopharyngeal carcinoma patient.

The present invention also includes the use of a reagent for detecting the gene mutation of *CCND1*, *FGF14*, *FGF3* or *FGF4* chromosome 11q13 region in preparing a kit for predicting the therapeutic effect of an anti-PD-1 antibody on a malignant cancer. Such agents include, but are not limited to, those conventionally used in assays, including but not limited to, primers, probes, agents required for PCR, etc.

The present invention also includes the use of a reagent for detecting the number of copies of EBV DNA in peripheral blood in preparing a kit for predicting the therapeutic effect of an anti-PD-1 antibody on a malignant cancer. Such agents include, but are not limited to, those conventionally used in assays, including but not limited to, primers, probes, agents required for PCR, etc.

### Medical kit

The present invention also provides a medical kit, which contains one or more single drug dosage units of the anti-PD-1 antibody or the antigen-binding fragment thereof as described in any of the embodiments herein; or contains one or more single drug dosage units of the anti-PD-1 antibody or the antigen-binding fragment thereof as described in any of the embodiments herein, one or more single drug dosage units of gemcitabine as described in any of the embodiments herein, and one or more single drug dosage units of cisplatin as described in any of the embodiments herein.

In some embodiments, the medical kit contains one or more single drug dosage units of the drug combination as described in any of the embodiments herein. In some embodiments, the medical kit contains one or more groups of pharmaceutical formulations, wherein each group of pharmaceutical formulations contains a three-week administration dose, including one dose of toripalimab, two doses of gemcitabine, and one dose of cisplatin; preferably, the monoclonal antibody toripalimab is at a fixed dose of about 240 mg; the two doses of gemcitabine are enough to be administered twice at a dose of about 1000 mg/m² body surface area; and the one dose of cisplatin is enough to be administered once at a dose of about 80 mg/m² body surface area.

The medical kit of the present invention can be used in treatment of a malignant cancer as described in any of the embodiments herein, especially nasopharyngeal carcinoma. Preferably, the amount of an anti-cancer active ingredient contained in the medical kit of the present invention is sufficient for at least one course of treatment (such as 2-8 courses of treatment) according to the treatment method as described herein. In the case of containing the drug combination of the present invention, the one course of treatment includes at least one administration of toripalimab, two administrations of gemcitabine and one administration of cisplatin.

### Abbreviation

The following abbreviations are used throughout the description and examples of the present invention:
BID Bis in die
CDR Complementarity determining region
DFS Disease-free survival
FR Framework region
IgG Immunoglobulin G
IHC Immunohistochemistry
OR Overall response
ORR Objective response rate
OS Overall survival
PD Progressive disease
PFS Progression-free survival
PR Partial response
CR Complete response
SD Stable disease
DLT Dose-limiting toxicity
MTD Maximum tolerated dose
AE Adverse event
Q2W Quaque 2 week/every 2 weeks
QD Quaque die/every day
CSD Chronic sun-induced damage
non-CSD Non-chronic sun-induced damage
IRC Independent review committee
TRAE Treatment-related adverse event
SAE Serious adverse event
RO Receptor occupancy
UC Urothelial carcinoma
RCC Renal cell carcinoma
MM Metastatic melanoma
RECIST Response Evaluation Criteria in Solid Tumour
irRECIST Immune-Related Response Evaluation Criteria in Solid Tumour
DOR Duration of response
MSI Microsatellite instability
BICR: Blinded Independent Centre Review

The present invention is further illustrated by the following examples, which should not be construed as limiting the present invention. The contents of all references cited throughout the present application are explicitly incorporated herein by reference.

### Examples

### Example 1: Clinical study on anti-PD-1 antibody for treating nasopharyngeal carcinoma

Inclusion criteria: eligible subjects must (1) be 18 years old or older, (2) have recurrent or metastatic nasopharyngeal carcinoma, (3) be refractory to standard systemic therapy or have progressive disease after 6 months of radiotherapy and chemotherapy, (4) have an ECOG score of 0 or 1, (5) have the normal organ function within 10 days after treatment, (6) have no history of autoimmune diseases or other malignant cancers, and (7) have not received any anti-PD-1/or anti-PD-L1 immunotherapy before.

Subjects must have an evaluable lesion according to RECIST v 1.1, have not received anti-tumour monoclonal antibody drug treatment within 4 weeks before treatment, have not received any anti-tumour drug treatment within 2 weeks before treatment, and have not received systemic steroid drug treatment within 7 days before treatment.

From December 22, 2016 to February 19, 2019, a total of 279 patients with recurrent or metastatic NPC were screened from 17 centres in Chinese mainland, and 190 patients were enrolled in this study. The patients had a mean age of 46.4 years, and most of them were male (n = 158, 83.2%). Of the two histological subtypes, 182 patients (95.8%) had non-keratinizing NPC and 8 patients (4.2%) had keratinizing NPC. There were 116 patients (61.1%) who had received at least two kinds of systemic therapy. The demographics of the enrolled subjects are shown in Table 1.

**Table 1: Demographics of the enrolled subjects**

| Characteristics | Description | Numerical value (100%) |
|---|---|---|
| Age | Number | 190 |
| | Mean | 46.4 |
| | Min, Max | 22.0, 71.0 |
| Gender | Male | 158 (83.2) |
| | Female | 32 (16.8) |
| ECOG | 0 | 66 (34.7) |
| | 1 | 124 (65.3) |
| TNM stage | III | 3 (1.6) |
| | IV 1a | 10 (5.3) |
| | IV 1b | 177 (93.1) |
| NPC subtype | Keratinizing | 8 (4.2) |
| | Non-keratinizing | 182 (95.8) |
| PD-L1 status* | Positive | 48 (25.3) |
| | Negative | 134 (70.5) |
| | Unknown | 8 (4.2) |
| Baseline LDH | > 2xULN | 35 (18.4) |
| | ≤ 2xULN | 154 (81.1) |
| | NA | 1 (0.5) |
| Early treatment line | 0 | 15 (7.9) |
| | 1 | 59 (31.0) |
| | 2+ | 116 (61.1) |
| Number of copies of EBV DNA | ≥ 10000 IU/mL | 104 (54.5) |
| | < 10000 IU/mL | 75 (39.5) |
| | N/A | 11 (5.8) |

| | | |
|---|---|---|
| Notes: *Positive PD-L1 is defined as PD-L1 expression ≥ 1% by staining of tumour cells with SP142 IHC. | | |

### Test drug: anti-PD-1 antibody toripalimab (WO 2014206107).

The enrolled subjects received 3 mg/kg toripalimab by intravenous injection once every two weeks (Q2W) until confirmed progressive disease, intolerable toxicity, individual's withdrawal of consent, investigator's decision to discontinue treatment, or the end of treatment of 24 months.

The subjects were evaluated every 8 weeks for the first year and every 12 weeks thereafter according to RECIST v1.1 and the Immune-Related Response Evaluation Criteria in Solid Tumour (irRECIST). The subjects were evaluated every 3 months after drug discontinuance.

### Clinical design:

This was a single-arm, phase II, open-label clinical trial. This study was conducted to evaluate the safety and anti-tumour activity of an anti-PD-1 antibody in treatment of a recurrent or metastatic nasopharyngeal carcinoma patient.

### 1.1 Safety study:

As of February 19, 2020 (i.e., 12 months after the last patient was admitted to the hospital), patients had received a mean of 8 doses of toripalimab (range: 1 to 69 doses). There were 181 patients (95.3%) experiencing treatment-emergent adverse events (TEAEs), of which 141 patients (74.2%) experienced treatment-related adverse events (TRAEs). The common TRAEs (> 5%) are shown in Table 2. There were 63 patients (33.2%) experiencing TEAEs of grade 3 or above, while 27 patients (14.2%) experienced TRAEs of grade 3 or above. Four patients (2.1%) discontinued drugs due to TRAEs, and seven patients (3.7%) were subjected to dose interruption due to TRAEs. Immune-related adverse events (AEs) included 45 cases (23.7%) of hypothyroidism, 5 cases (2.6%) of hyperthyroidism, 3 cases (1.6%) of liver dysfunction, 3 cases (1.6%) of interstitial lung disease, 1 case (0.5%) of dermatomyositis and 1 case (0.5%) of autoimmune myocarditis.

**Table 2: Common > 5%) adverse events related to treatment with toripalimab (n = 190)**

| N (%) | All | Grade 1 | Grade 2 | Grade 3 | Grade 4 | Grade 5 |
|---|---|---|---|---|---|---|
| All adverse events | 141 (74.2) | 55 (28.9) | 59 (31.1) | 17 (8.9) | 4 (2.1) | 6 (3.2) |
| Hypothyroidism | 45 (23.7) | 19 (10.0) | 26 (13.7) | 0 | 0 | 0 |
| Anaemia | 29 (15.3) | 15 (7.9) | 12 (6.3) | 2 (1.1) | 0 | 0 |
| Elevated AST | 29 (15.3) | 26 (13.7) | 3 (1.6) | 0 | 0 | 0 |
| Elevated ALT | 26 (13.7) | 21(11.1) | 5 (2.6) | 0 | 0 | 0 |
| Fatigue | 25 (13.2) | 18 (9.5) | 5 (2.6) | 2 (1.1) | 0 | 0 |
| Proteinuria | 24 (12.6) | 24 (12.6) | 0 | 0 | 0 | 0 |
| Leukopenia | 19 (10.0) | 8 (4.2) | 11 (5.8) | 0 | 0 | 0 |
| Fever | 18 (9.5) | 13 (6.8) | 5 (2.6) | 0 | 0 | 0 |
| Pruritus | 16 (8.4) | 14 (7.4) | 2(1.1) | 0 | 0 | 0 |
| Rash | 12 (6.3) | 8 (4.2) | 4 (2.1) | 0 | 0 | 0 |
| Neutropenia | 10 (5.3) | 6 (3.2) | 3 (1.6) | 1 (0.5) | 0 | 0 |

### 1.2 Anti-tumour activity study:

As of February 19, 2020, among all 190 patients, 94 patients (49.5%) had died, 78 patients (41.1%) had discontinued drugs, and 18 patients (9.5%) had remained on study. The patients were treated for a median of 3.7 months (0.2 month to 34.8 months). Among the 190 patients evaluated by IRCIST/RECIST v1.1, the objective response rate (ORR) was 20.5% (95% CI: 15.0-27.0), including 5 cases of CR, 34 cases of PR and 37 cases of SD, and the disease control rate (DCR) was 40.0% (95% CI: 33.0-47.3). As evaluated by IRC/irRECIST, ORR was 20.5% (95% CI: 15.0-27.0) and DCR was 47.9% (95% CI: 40.6-55.2) (Table 3).

**Table 3: Evaluation of clinical efficacy according to RECIST v1.1 or irRECIST**

| | IRC | | Investigator | |
|---|---|---|---|---|
| | RECIST 1.1 | irRECIST | RECIST1.1 | irRECIST |
| CR | 5 (2.6%) | 5 (2.6%) | 5 (2.6%) | 5 (2.6%) |
| PR | 34 (17.9%) | 34 (17.9%) | 35 (18.4%) | 39 (20.5%) |
| SD | 37 (19.5%) | 52 (27.4%) | 48 (25.3%) | 59 (31.1%) |
| PD | 86 (45.3%) | 71 (37.4%) | 78 (41.1%) | 59 (31.1%) |
| NE | 28 (14.7%) | 28 (14.7%) | 24 (12.6%) | 28 (14.7%) |
| Total | 190 (100.0%) | 190 (100.0%) | 190 (100.0%) | 190 (100.0%) |
| ORR (%)* | 39 (20.5%) | 39 (20.5%) | 40 (21.1%) | 44 (23.2%) |
| 95% CI | 15.0%, 27.0% | 15.0%, 27.0% | 15.5%, 27.5% | 17.4%, 29.8% |
| DCR (%)** | 76 (40.0%) | 91 (47.9%) | 88 (46.3%) | 103 (54.2%) |
| 95% CI | 33.0%, 47.3% | 40.6%, 55.2% | 39.1%, 53.7% | 46.8%, 61.4% |

| | | | | |
|---|---|---|---|---|
| Notes: *ORR = (CR + PR)/total number × 100%; **DCR = (CR + PR + SD)/total number × 100%; | | | | |

CR: Complete response; PR: Partial response; SD: Stable disease; PD: Progressive disease; NE: Not evaluated; ORR: Objective response rate; DCR: Disease control rate; CI: Confidence interval.

There were 73 subjects (38.4%) who had reductions in target lesions relative to baseline, of which 48 subjects (25.3%) had a reduction greater than or equal to 30% in target lesions from baseline (FIGs. 1a and 1b). The subjects had a median time to response of 1.8 months (95% CI: 1.8-2.1); a median progression-free survival (mPFS) of 1.9 months (95% CI: 1.8-3.5) (FIG. 2a); a median overall survival (mOS) of 17.4 months (95% CI: 11.7-22.9) (FIG. 2b); and a median DOR of 12.8 months (95% CI: 9.4-NE), which indicates a lasting response (FIG. 2c). Patients who had received at least two prior treatments (n = 116) had an ORR of 21.6% (95% CI: 14.5-30.1), a median DOR of 21.5 months, mPFS of 2.0 months, and mOS of 15.1 months.

Since only 7 PR/CR subjects and 11 SD subjects died, the median OS for subjects experiencing objective response (n = 39) or stable disease (n = 38) was not reached. Subjects with progressive disease (n = 113) had a median OS of 8.4 months.

### 1.3 Immunogenicity

Anti-drug antibodies (ADAs) were detected in 190 patients. Seven cases (3.7%) were positive for ADA, including four consecutive positive samples. There was no significant difference in the incidence of AEs, SAEs and AEs of grade 3 or above, drug discontinuance or dose delay, and clinical efficacy between ADA positive and negative patients.

### 1.4 Histological subtypes

As evaluated by IRC, patients with keratinizing NPC (n = 8) had a significantly better ORR than patients with non-keratinizing NPC (n = 182), i.e., 62.5% versus 18.7% (*p* = 0.01). In addition, patients with keratinizing NPC had an obviously better PFS than patients with non-keratinizing NPC, i.e., 16.6 months versus 1.9 months (HR = 0.46 (95% CI: 0.25-0.85),*p* = 0.013). OS was not reached versus was 15.1 months, and the difference was not statistically significant (HR = 0.51 (95% CI: 0.21-1.24), *p* = 0.14) (FIGs. 2d and 2e).

### Example 2: Study on correlation between biomarkers and clinical efficacy

### 2.1 PD-L1 expression in tumour

The PD-L1 expression status in tumour biopsies was determined by SP142 IHC staining, with tumour proportion score (TPS) > 1% being determined as positive. Among the 190 patients, 48 patients (25.3%) were PD-L1 positive and 134 patients (70.5%) were PD-L1 negative (FIG. 3a). Eight patients (4.2%) had unknown PD-L1 expression status. Among the PD-L1+ patients, 21 patients (11.1%) were identified as having high PD-L1 expression (> 25%). According to histological subtypes, patients with keratinizing NPC (75.0%) had a significantly higher percentage of PD-L1+ than patients with non-keratinizing NPC (24.1%) (*p* = 0.0047). The PD-L1+ patients had higher ORR values than PD-L1- patients, with ORR being 27.1% versus 19.4%, but the difference was not statistically significant (*p* = 0.31). In patients with PD-L1 > 25%, the difference in ORR was more significant (38.1% versus 19.3%, *p* value = 0.08) (FIG. 3a). Patients with PD-L1 > 25% also had better PFS and OS than those with PD-L1 ≤ 25%, with mPFS being 7.2 months versus 1.9 months (HR = 0.64 (95% CI: 0.40-1.02), *p* = 0.059); mOS being not reached versus 15.1 months (HR = 0.57 (95% CI: 0.31-1.05), *p* = 0.071) (FIGs. 3b and 3c), but the difference was not statistically significant.

### 2.2 Analysis of tumour mutation load (TMB)

Tumour biopsy tissues and matched peripheral blood samples from subjects were subjected to whole exome sequencing (WES) to identify tumour-specific mutations. Valid WES results were available for 174 patients. The NPC patients had very low TMB with a median TMB of 0.95 mutations per million base pairs (Muts/Mb). Only one patient had high MSI, four patients had more than 10 mutations/Mb in TMB, and the remaining patients had no more than 5.8 mutations/Mb in TMB. In this study, the top 10% or 20% cut-off values of TMB (2.9 Muts/Mb and 2.0 Muts/Mb, respectively) were selected for evaluation of clinical response. The ORRs for the top 10% and 20% of patients were 17.6% and 14.3%, respectively (FIG. 3a). Four patients with TMB more than 10 Muts/Mb, including one patient with high MSI, progressed to the best responders. In addition, at 1.9 months, the 10% of patients with the highest TMB value had a similar PFS to the 90% of patients with the lowest TMB value (FIG. 3d). On the contrary, high TMB patients had lower OS values than low TMB patients, i.e., 9.2 months versus 17.4 months, but the difference was not statistically significant (FIG. 3e). To sum up, in this study, TMB was not associated with clinical response in patients with advanced NPC who received single drug therapy of toripalimab.

### 2.3 Analysis of genome mutation

The most frequently changed genes (≥ 10%) were identified by WES, including *CDKN2A (20%), TP53* (13%), *NFKB1A* (13%), *CDKN2B* (11%), *ETV6* (11%) and *MCL1* (10%) (FIG. 4). The correlation between genomic changes and clinical efficacy was analysed. The study found that 11 patients who had a genome amplification of *CCND1* (n = 11) and/or *FGF14, FGF3* or *FGF4* chromosome 11q13 region and were treated with toripalimab had an ORR of 0%. There were 19 patients with *ETV6* mutations, and the ORR was only 5.3%.

### 2.4 Number of copies of EBV DNA in plasma

Plasma was collected from patients before administration, and the number of copies of EBV DNA was analysed by qRT-PCR once every 4 weeks. Patients with baseline EBV titre < 10000 IU/mL had higher ORR than patients with EBV titre ≥ 10000 IU/mL, i.e., 26.7% versus 15.4% (*p* = 0.088). The number of copies of EBV DNA in dynamic plasma during treatment was collected from 149 patients (FIG. 5). The study found a median 31-fold decrease in the number of copies of EBV DNA in plasma from baseline to the nadir number of copies in patients with objective response (n = 34), a 3-fold decrease in patients with stable disease (n = 35), and no change in patients with progressive disease (n = 80) (FIG. 5). In addition, on day 28 (namely two weeks before the first radiologic evaluation of clinical activities), patients with at least 2-fold decrease in the number of copies of EBV DNA in plasma (n = 60) had significantly better clinical response rate than those with less than 2-fold decrease (n = 88), with ORR being 48.3% versus 5.7% (*p* = 0.0001). In contrast, 14 patients who experienced progressive disease had a median of 3 months and at least 2-fold increase in the number of copies of EBV DNA in plasma, prior to the imaging-confirmed progressive disease (FIG. 5).

### Example 3: Clinical study on anti-PD-1 antibody combined with gemcitabine-cisplatin (GP) for treating nasopharyngeal carcinoma

### Subjects

Inclusion criteria: eligible subjects must meet the following criteria: being 18-75 years old; suffering from recurrent or metastatic nasopharyngeal carcinoma (if conforming to the definition of the eighth edition of NPC staging system of the Union for International Cancer Control and the American Joint Committee on Cancer, the nasopharyngeal carcinoma is in stage IVB); having not received systemic chemotherapy for recurrent or metastatic diseases before; having at least one measurable lesion according to RECIST v1.1; having a life expectancy of more than 3 months; having a performance status score of the Eastern Cooperative Oncology Group of 0 or 1; having the normal organ function. For NPC that recurred after systemic therapy, according to the Common Terminology Criteria of National Cancer Institute, the interval between recurrence and the last dose of previous radiotherapy or chemotherapy must be more than 6 months, and any prior treatment toxicity must have been reduced to grade 0 or grade 1 (CTCAE v5.0).

Exclusion criteria include: having a history of severe hypersensitivity to any component of any monoclonal antibody, such as gemcitabine, cisplatin or toripalimab; active or untreated central nervous system metastasis or spinal cord compression; potential risk of massive haemorrhage due to necrotic lesions; uncontrolled pleural or pericardial effusion; uncontrolled ascites; uncontrolled tumour-related pain; uncontrolled or symptomatic hypercalcemia; previous malignant cancers other than NPC in the past 5 years, excluding those with minimal risk of metastasis or death after expected treatment; using a monoclonal antibody PD-1/PD-L1/CTLA4 therapy before treatment; using traditional anti-tumour herbs in the past 4 weeks; having a history of major surgery in the past 28 days, or being expected to have major surgery during the study; having a history of autoimmune diseases; using systemic immunostimulants within 4 weeks before treatment or within the half-life of the drug; using systemic corticosteroids or immunosuppressive drugs within 2 weeks before treatment; having a history of bone marrow or solid organ transplantation; having a history of non-infectious pneumonia or current pneumonia; using any live vaccine within the first 4 weeks; active tuberculosis, active hepatitis B virus or hepatitis C virus infection; active human immunodeficiency virus; active major neuropathy or psychosis; peripheral neuropathy of grade 2 or above; pregnant or lactating women or clinically important cardiovascular diseases.

### Test drug

Anti-PD-1 antibody: toripalimab, Suzhou Junmeng Biosciences Co., Ltd.; gemcitabine/Gem, and cisplatin/Cis

### Clinical design

This was a randomized, double-blind and placebo-controlled study. Randomization was carried out using the Interactive VoiceWeb Response System (IVRS). Before enrolment, patients were grouped according to their ECOG performance status (0 or 1) and disease stage (local recurrence and primary metastasis).

The subjects were randomly divided into group A and group B with a ratio of 1:1. Group A received toripalimab combined with gemcitabine (Gem) and cisplatin (Cis) once every 3 weeks (Q3W), and group B received placebo combined with gemcitabine and cisplatin once every 3 weeks (Q3W). All drugs were administered by intravenous infusion. Patients would receive toripalimab (240 mg) or placebo on day 1 of each 3-week cycle, gemcitabine (1000 mg/m² body surface area) on days 1 and 8, and cisplatin (80 mg/m² body surface area) on day 1. Chemotherapy would continue until progressive disease, intolerable toxicity, non-compliance, withdrawal of consent or a maximum of 6 cycles, whichever occurred first during chemotherapy. In the maintenance stage, patients would receive toripalimab (240 mg) (group A) or placebo (group B) every 3 weeks as a maintenance therapy until progressive disease, intolerable toxicity, withdrawal of consent or investigator's judgment or up to 2 years. Crossover experiments were not allowed as a part of the study.

### Endpoint

The primary endpoint was progression-free survival (PFS) in the intention-to-treat population, defined as the time from randomization to first documented progressive disease or death from any cause, whichever occurred first. The secondary endpoints included overall survival (OS); objective response rate (ORR), defined as the proportion of patients with confirmed complete response or partial response; duration of response (DoR), defined as the time between first documented response and first evidence of progressive disease; disease control rate (DCR), defined as the proportion of patients with complete response (CR) or partial response (PR) or stable disease (SD) as the best response; 1-year and 2-year PFS rates and OS rates in the ITT population.

### Evaluation

The baseline tumour evaluation included CT scans of the nasopharynx, neck, chest and abdomen (oral contrast/venography unless contraindicated) or MRI or systemic positron emission tomography (PET)/CT scans. If there were clinical indications, bone scanning should be performed. All known disease sites were documented at screening and re-evaluated at each subsequent tumour evaluation. Throughout the study, the same radiographic procedure as that used for evaluating the baseline disease site was used. Tumours were evaluated every 6 weeks for the first 12 months and then every 9 weeks thereafter until progressive disease, loss of clinical benefits, withdrawal of consent, initiation of a new anticancer therapy, death or study termination by the investigator (whichever occurred first).

According to RECIST v1.1 and irRECIST, the investigator and Blinded Independent Centre Review (BICR) evaluated the clinical response.

### 3.1 Treatment of subjects

From November 10, 2018 to October 20, 2019, 408 subjects were screened from 48 sites in China and Singapore (FIG. 6). A total of 289 eligible subjects were randomly assigned to a toripalimab-combination group (group A, n = 146) or a placebo-combination group (group B, n = 143). Although the toripalimab group had a higher proportion of smokers and drinkers than the placebo group, the baseline demographics and disease characteristics were generally balanced between the two groups (52.1% vs. 41.3%, P = 0.077 and 20.5% vs. 12.6%, P = 0.082). However, the difference was not statistically significant. There were 74.7% of subjects from the toripalimab group and 76.2% of subjects from the placebo group who were stained positive for PD-L1 expression, as defined by >_ 1% of positive tumour cells or immune cells. The demographics of the enrolled subjects are shown in Table 4.

During chemotherapy, all patients received at least one dose of the study drug, and 56 subjects (19.4%) had discontinued treatment study by the cut-off date (31 subjects in the toripalimab group and 25 subjects in the placebo group). Both groups received 6 cycles of chemotherapy. After the completion of chemotherapy, 231 patients (79.9%) continued to receive a maintenance therapy (113 patients in the toripalimab group and 118 patients in the placebo group). Subjects received an average of 12 courses of toripalimab treatment and 11 courses of placebo treatment.

**Table 4: Demographics of the enrolled subjects**

| Characteristics | Toripalimab + Gem/Cis | Placebo + Gem/Cis | Total |
|---|---|---|---|
| | (N = 146) | (N = 143) | (N = 289) |
| Age | | | |
| Median | 46 | 51 | 48 |
| Range | 19-72 | 21-72 | 19-72 |
| Gender (%) | | | |
| Female | 22 (15) | 27 (19) | 49 (17) |
| Male | 124 (85) | 116 (81) | 240 (83) |

| Race (%) | | | |
|---|---|---|---|
| Asia | 146 (100) | 143 (100) | 289 (100) |
| Others | 0(0) | 0(0) | 0(0) |

| ECOG score (%) | | | |
|---|---|---|---|
| 0 | 83 (57) | 81 (57) | 164 (57) |
| 1 | 63 (43) | 62 (43) | 125 (43) |

| Disease status (%) | | | |
|---|---|---|---|
| Recurrent/metastatic after treatment | 83 (57) | 82 (57) | 165 (57) |
| Metastatic | 63 (43) | 61 (43) | 124 (43) |

| Smoking status (%) | | | |
|---|---|---|---|
| Current or former smoker | 76 (52) | 59 (41) | 135 (47) |
| Non-smoker | 70 (48) | 84 (59) | 154 (53) |

| Histology (%) | | | |
|---|---|---|---|
| Non-keratinizing squamous cell carcinoma | 145 (99) | 141 (99) | 285 (99) |
| Keratinizing squamous cell carcinoma | 1 (1) | 2(1) | 3(1) |
| Others | 1 (1) | 0(0) | 1 (0) |
| Surgery before treatment (%) | 38 (26) | 43 (30) | 81 (28) |
| Radiotherapy before treatment (%) | 85 (58) | 87 (61) | 172 (60) |
| New auxiliary systemic therapy before treatment (%) | 54 (37) | 49 (34) | 103 (36) |
| Systemic radiotherapy before treatment (%) | 65 (45) | 68 (48) | 133 (46) |
| Auxiliary systemic therapy before treatment (%) | 24 (16) | 21 (15) | 45 (16) |

### 3.2 Progression-free survival

As evaluated by BICR according to RECIST v1.1, in a pre-specified interim analysis of 128 events of progressive disease or death, the toripalimab and placebo groups had a median duration of treatment of 39 weeks and 36 weeks, respectively. The toripalimab group had a median PFS of 11.7 months (95% CI, 11.0 to NE) versus 8.0 months for the placebo group (95% CI, 7.0 to 9.5). The toripalimab group had a significant improvement in PFS compared with placebo (the hazard ratio for progression or death: 0.52; 95% CI: 0.36 to 0.74; two-sided P = 0.0003) (FIG. 6a). The toripalimab group had an estimated 1-year PFS of 49.4% (95% CI: 36.4 to 61.1) versus 27.9% (95% CI: 18.0 to 38.8) for the placebo group, with a difference of 21.4% (95% CI: 5.1 to 37.8). In all relevant subgroups, including all PD-L1 subgroups, it was observed that the toripalimab group had a better PFS than placebo (FIG. 6c). For patients with PD-L1 positive tumour cells (TC) >_ 1% or immune cells (IC) >_ 1%, the hazard ratio for progression or death between the toripalimab group and the placebo group was 0.59 (95% CI: 0.39 to 0.89), and the median PFS was 11.4 months versus 8.2 months. For patients with PD-L1 positive tumour cells (TC) < 1% and immune cells (IC) < 1%, the hazard ratio for progression or death was 0.35 (95% CI: 0.15 to 0.81), and the median PFS was 11.0 months versus 6.0 months.

As evaluated by investigators according to RECIST v1.1, treatment with toripalimab combined with chemotherapy could reduce the risk of progression or death by 59% compared with placebo combined with chemotherapy (HR = 0.41; 95% CI: 0.28 to 0.59; P < 0.0001) (FIG. 6b). The toripalimab group had a 1-year PFS rate of 59.5% versus 20.0% for the placebo group, with a difference of 39.5% (95% CI: 25.6 to 53.5).

### 3.3 Overall survival

By the cutoff date of the interim analysis of May 30, 2020, 29 deaths were reported, including 12 deaths (8.2%) in the toripalimab group and 17 deaths (11.9%) in the placebo group. The median survival was not reached in both groups. The stratified hazard ratio for OS was 0.78 (95% CI: 0.37 to 1.64; P = 0.50). According to the survival update on February 18, 2021, a total of 64 deaths were reported, including 25 deaths in the toripalimab group and 39 deaths in the placebo group. The stratified hazard ratio for OS was 0.60 (95% CI: 0.36 to 1.00; P = 0.0462), and patients in the toripalimab group had a 40% reduction in the risk of immediate death compared with those in the placebo group (FIG. 7). The toripalimab group had an estimated 2-year patient survival rate of 77.8% (95% CI: 68.0 to 85.0) versus 63.3% for the placebo group (95% CI: 49.8 to 74.1). Due to the limited number of OS events, the median OS was immature for either group.

### 3.4 Tumour response

As evaluated by BICR, 28 patients (19.2%) in the toripalimab group and 16 patients (11.2%) in the placebo group had confirmed complete response; 85 patients (58.2%) in the toripalimab group and 79 patients (55.2%) in the placebo group had confirmed partial response. The toripalimab group had an ORR of 77.4% (95% CI: 69.8 to 83.9) versus 66.4% for the placebo group (95% CI: 58.1 to 74.1) (P = 0.0335). The toripalimab group had a DCR of 87.7% (95% CI: 81.2 to 92.5) versus 79.7% for the placebo group (95% CI: 72.2 to 86.0) (P = 0.0650) (Table 5). As evaluated by investigators, the toripalimab group had an ORR of 80.8% (95% CI: 73.5 to 86.9) versus 74.8% for the placebo group (95% CI: 66.9 to 81.7).

As evaluated by BICR in the ITT population, 114 patients in the toripalimab group and 95 patients in the placebo group were responders. The toripalimab group had a median DoR of 10.0 months (95% CI: 8.8 to NE) versus 5.7 months for the placebo group (95% CI: 5.4 to 6.8) (HR: 0.50; 95% CI: 0.33 to 0.78) (Table 5). As of May 30, 2020, responses were ongoing in 66% (75/114) of the toripalimab group and 43% (41/95) of the placebo group (FIG. 8). As evaluated by investigators in the ITT population, the median DoR was not reached in the toripalimab group (95% CI: 9.7 to NE), and was 5.8 months (95% CI: 5.7 to 6.9) in the placebo group (HR: 0.37; 95% CI: 0.24 to 0.56).

**Table 5: Tumour response with toripalimab and placebo combined with gemcitabine and cisplatin in patients with recurrent or metastatic nasopharyngeal carcinoma according to RECIST v1.1**

| Variable | | Toripalimab + Gem/Cis | Placebo + Gem/Cis |
|---|---|---|---|
| | | (N = 146) | (N = 143) |
| Objective response | | | |
| Number of patients | | 115 | 95 |
| | Objective response rate % (95% CI) | 79 (71-85) | 66 (58-74) |
| | Difference in objective response rate % (95% CI) | 12 (2-22) | |
| | P-value | 0.0149 | |

| Best overall response rate (%) | | | |
|---|---|---|---|
| | Complete response | 28(19) | 16 (11) |
| | Partial response | 87 (60) | 79 (55) |
| | Stable disease | 16 (11) | 19 (13) |
| | Progressive disease | 4 (3) | 8(6) |
| | Unevaluable | 7 (5) | 8 (6) |
| | Non-CR/non-PD | 4 (3) | 12 (8) |
| | No disease certificate | 0(0) | 1 (1) |

| Duration of response - month | | | |
|---|---|---|---|
| | Median (95% CI) | 10.0 (8.8-NE) | 5.7 (5.4-6.8) |
| | P-value | 0.0014 | |

### 3.5 Adverse events

As of May 30, 2020, the toripalimab group had a median treatment duration of 38.7 weeks versus 36.0 weeks for the placebo group. In the toripalimab group and the placebo group, the median exposure to cisplatin was 18.3 and 18.4 weeks, respectively, and the median exposure to gemcitabine was 19.3 and 19.6 weeks, respectively. All patients experienced at least one treatment-emergent adverse event (TEAE). The incidence of TEAEs ≥ 3 in the toripalimab group and the placebo group was 89.0% and 89.5%, respectively. The proportion of toripalimab/placebo discontinuation due to TEAEs in both groups was 7.5% and 4.9%, respectively. Serious adverse events (SAEs) (41.1% vs. 43.4%) and fatal TEAEs (2.7% vs. 2.8%) were similar in both groups.

The most common TEAEs included leukopenia (91.1% in the toripalimab group vs. 94.4% in the placebo group), anaemia (88.4% vs. 94.4%), neutropenia (85.6% vs. 93.0%), nausea (69.2% vs. 83.2%), vomiting (67.1% vs. 65.7%), thrombocytopenia (63.0% vs. 62.2%), and loss of appetite (53.4% vs. 58.7%).

The incidence of TEAEs of grade 3 or above was similar in both groups, including leukopenia (61.6% vs. 58.0%), neutropenia (57.5% vs. 63.6%), anaemia (47.3% vs. 39.9%), thrombocytopenia (32.9% vs. 28.7%), pneumonia (10.3% vs. 3.5%), lymphopenia (8.9% vs. 7.0%), hyponatremia (8.9% vs. 4.2%), and hypokalaemia (6.8% vs. 7.0%).

### 3.6 Conclusion

In this randomized phase III study, we compared the efficacy and toxicity of toripalimab and placebo combined with GP (gemcitabine and cisplatin) chemotherapy in treatment of recurrent or metastatic NPC. The results showed that compared with chemotherapy plus placebo, the addition of toripalimab in chemotherapy could provide higher overall response and longer overall survival, with a manageable safety profile.

## Claims

1. Use of an anti-PD-1 antibody or an antigen-binding fragment thereof in preparing a drug for preventing or treating a malignant cancer, and use of a combination of an anti-PD-1 antibody or an antigen-binding fragment thereof and gemcitabine-cisplatin in preparing a drug for preventing or treating a malignant cancer, wherein preferably, the malignant cancer is nasopharyngeal carcinoma; and more preferably, the malignant cancer is recurrent or metastatic nasopharyngeal carcinoma.

2. The use of claim 1, **characterised in that** the malignant cancer is a malignant cancer with PD-L1 expression > 1% in a cancer tissue section by immunohistochemical staining analysis, preferably a malignant cancer with PD-L1 expression > 25% in a cancer tissue section by immunohistochemical staining analysis.

3. The use of claim 1, **characterised in that** the malignant cancer is selected from keratinizing nasopharyngeal carcinoma and non-keratinizing nasopharyngeal carcinoma, preferably keratinizing nasopharyngeal carcinoma.

4. The use of claim 1, **characterised in that** the nasopharyngeal carcinoma is a nasopharyngeal carcinoma in which no genome amplification of *CCND1*, *FGF14*, *FGF3* or *FGF4* chromosome 1 1q13 region is detected in peripheral blood circulating tumour DNA or in a tumour tissue, or
the nasopharyngeal carcinoma is a nasopharyngeal carcinoma in which there is at least 2-fold decrease in the number of copies of EBV DNA in peripheral blood on day 28 of treatment relative to before administration on day 0.

5. The use of any one of claims 1-4, **characterised in that** the anti-PD-1 antibody or the antigen-binding fragment thereof comprises light chain complementarity determining regions with amino acid sequences as set forth in SEQ ID NOs: 1, 2 and 3, and heavy chain complementarity determining regions with amino acid sequences as set forth in SEQ ID NOs: 4, 5 and 6; preferably, the anti-PD-1 antibody or the antigen-binding fragment thereof comprises a light chain variable region with an amino acid sequence as set forth in SEQ ID NO: 7, and a heavy chain variable region with an amino acid sequence as set forth in SEQ ID NO: 8; and more preferably, the anti-PD-1 antibody comprises a light chain with an amino acid sequence as set forth in SEQ ID NO: 9, and a heavy chain with an amino acid sequence as set forth in SEQ ID NO: 10.

6. The use of any one of claims 1-5, **characterised in that** the anti-PD-1 antibody is selected from one or more of nivolumab, pembrolizumab, toripalimab, sintilimab, camrelizumab, tislelizumab and cemiplimab, preferably toripalimab.

7. The use of any one of claims 1-6, **characterised in that**
the anti-PD-1 antibody or the antigen-binding fragment thereof is administered alone, wherein the anti-PD-1 antibody or the antigen-binding fragment thereof is administered at a dose from about 0.1 mg/kg body weight to about 10.0 mg/kg body weight of an individual, such as about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight of an individual, or at a dose selected from a fixed dose of about 120 mg to about 480 mg, such as a fixed dose of 120 mg, 240 mg, 360 mg or 480 mg, preferably 3 mg/kg body weight of an individual or a fixed dose of 240 mg; or
the anti-PD-1 antibody or the antigen-binding fragment thereof is administered in combination with gemcitabine and cisplatin, wherein the anti-PD-1 antibody or the antigen-binding fragment thereof is administered at a dose from about 0.1 mg/kg body weight to about 10.0 mg/kg body weight of an individual, such as about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight of an individual, or at a dose selected from a fixed dose of about 120 mg to about 480 mg, such as a fixed dose of 120 mg, 240 mg, 360 mg or 480 mg, preferably 3 mg/kg body weight of an individual or a fixed dose of 240 mg; the gemcitabine is administered at a single dose from about 600 mg/m² body surface area to about 1400 mg/m² body surface area, such as 800 mg/m² body surface area, 1000 mg/m² body surface area or 1200 mg/m² body surface area; and the cisplatin is administered at a single dose from about 40 mg/m² body surface area to about 120 mg/m² body surface area, such as 60 mg/m² body surface area, 80 mg/m² body surface area or 100 mg/m² body surface area.

8. The use of claim 7, **characterised in that**
the anti-PD-1 antibody or the antigen-binding fragment thereof is administered alone, wherein the anti-PD-1 antibody or the antigen-binding fragment thereof is administered at a frequency of about once every week, once every two weeks, once every three weeks, once every four weeks or once a month, preferably once every two weeks; or
the anti-PD-1 antibody or the antigen-binding fragment thereof is administered in combination with gemcitabine and cisplatin, wherein the anti-PD-1 antibody or the antigen-binding fragment thereof is administered at a frequency of about once every week, once every two weeks, once every three weeks, once every four weeks or once a month, preferably once every three weeks; and the gemcitabine is administered at a frequency of about once every week, once every two weeks, once every three weeks, twice every three weeks, once every four weeks or once a month, preferably twice every three weeks; and the cisplatin is administered at a frequency of about once every week, once every two weeks, once every three weeks, once every four weeks or once a month, preferably once every three weeks.

9. The use of claim 8, **characterised in that**
the anti-PD-1 antibody or the antigen-binding fragment thereof is administered alone, wherein the anti-PD-1 antibody or the antigen-binding fragment thereof is administered at a dose of 1 mg/kg body weight of an individual, 3 mg/kg body weight of an individual or 10 mg/kg body weight of an individual, or at a fixed dose of 240 mg or 480 mg, once every two weeks or once every three weeks; or
the anti-PD-1 antibody or the antigen-binding fragment thereof is administered in combination with gemcitabine and cisplatin, wherein the anti-PD-1 antibody or the antigen-binding fragment thereof is administered at a fixed dose of 240 mg, once every three weeks; and the gemcitabine is administered at a single dose of about 1000 mg/m² body surface area, twice every three weeks; and the cisplatin is administered at a single dose of about 80 mg/m² body surface area, once every three weeks.

10. The use of claim 9, **characterised in that** the anti-PD-1 antibody or the antigen-binding fragment thereof, gemcitabine and cisplatin are administered parenterally, e.g., by intravenous infusion, in a liquid dosage form, e.g., an injection.

11. The use of claim 10, **characterised in that** the anti-PD-1 antibody or the antigen-binding fragment thereof, gemcitabine and cisplatin are administered in periods of one week, two weeks, three weeks, one month, two months, three months, four months, five months, half a year or longer, respectively; and optionally, each administration period lasts for the identical or different period of time and is at identical or different intervals.

12. A drug combination comprising an anti-PD-1 antibody or an antigen-binding fragment thereof, gemcitabine and cisplatin, wherein preferably, the anti-PD-1 antibody or the antigen-binding fragment thereof comprises light chain complementarity determining regions with amino acid sequences as set forth in SEQ ID NOs: 1, 2 and 3, and heavy chain complementarity determining regions with amino acid sequences as set forth in SEQ ID NOs: 4, 5 and 6; preferably, the anti-PD-1 antibody or the antigen-binding fragment thereof comprises a light chain variable region with an amino acid sequence as set forth in SEQ ID NO: 7, and a heavy chain variable region with an amino acid sequence as set forth in SEQ ID NO: 8; preferably, the anti-PD-1 antibody comprises a light chain with an amino acid sequence as set forth in SEQ ID NO: 9, and a heavy chain with an amino acid sequence as set forth in SEQ ID NO: 10; and more preferably, the anti-PD-1 antibody is toripalimab.

13. A method for preventing or treating a malignant cancer, the method comprising
administering an effective amount of the anti-PD-1 antibody or the antigen-binding fragment thereof of either claim 5 or claim 6 to an individual in need thereof, wherein preferably, the anti PD-1 antibody or the antigen-binding fragment thereof is administered at a dose and a regimen as described in any one of claims 7-11; or
administering the drug combination of claim 12, wherein preferably, the anti-PD-1 antibody or the antigen-binding fragment thereof, gemcitabine and cisplatin in the drug combination are administered at a dose or a regimen as described in any one of claims 7-11;
and preferably, the malignant cancer is nasopharyngeal carcinoma, more preferably recurrent or metastatic nasopharyngeal carcinoma, or the malignant cancer is as described in claim 3 or 4.

14. Use of a reagent for detecting a gene mutation or amplification of *CCND1*, *FGF14*, *FGF3* or *FGF4* chromosome 11q13 region in peripheral blood circulating tumour DNA and/or in a tumour tissue of an individual and/or a reagent for detecting the number of copies of EBV DNA in peripheral blood of an individual in preparing a kit for predicting a therapeutic effect of an anti-PD-1 antibody or an antigen-binding fragment thereof on nasopharyngeal carcinoma, wherein preferably, the anti-PD-1 antibody or the antigen-binding fragment thereof is as described in claim 5 or 6.

15. A medical kit comprising:
one or more single drug dosage units of an anti-PD-1 antibody or an antigen-binding fragment thereof, wherein the anti-PD-1 antibody or the antigen-binding fragment thereof is as described in either claim 5 or claim 6; or
one or more single drug dosage units of an anti-PD-1 antibody or an antigen-binding fragment thereof, one or more single drug dosage units of gemcitabine and one or more single drug dosage units of cisplatin, wherein preferably, the anti-PD-1 antibody or the antigen-binding fragment thereof is as described in either claim 5 or claim 6; or
one or more single drug dosage units of the drug combination of claim 12.
